# EUROPEAN PATENT SPECIFICATION

(11) **EP 4 153 259 B1**
(45) Date of publication and mention of the grant of the patent: **31.12.2025**
(21) Application number: 20936540.2
(22) Date of filing: 13.11.2020
(51) Int. Cl.: A61L 27/56, A61L 27/18, A61F 2/34, A61F 2/30, A61L 27/58

(54) **HIP LABRUM SCAFFOLDS**
HÜFTLABRUMGERÜSTE
ÉCHAFAUDAGES DE LABRUM DE HANCHE

(30) Priority: 22.05.2020 US 202063029326 P
(43) Date of publication of application: 29.03.2023
(73) Proprietor: Orteq B.V., 3971 NG Driebergen-Rijsenburg (NL)
(72) Inventor: COLES, Simon, Bristol BS31 3JP (GB); ELSER, Christoph, 85665 Moosach (DE); SHIN, Michael, Kingston Upon Thames KT1 2LE (GB); OBERBECK, Christian L., Southampton, NY 11968 (US)
(74) Representative: Hoffmann Eitle
(86) International application number: PCT/US2020/060344
(87) International publication number: WO 2021/236151

(56) References cited:
- WO-A1-2015/134028
- JP-A- 2000 264 991
- US-A1- 2011 105 635
- US-A1- 2019 269 816
- US-B2- 8 691 542

## Description

### RELATED APPLICATIONS

The present application claims priority under 35 U.S.C. § 119(e) to U.S. Provisional Application Serial No. 63/029,326, filed May 22, 2020.

### FIELD

Hip labrum scaffolds are generally provided.

### BACKGROUND

Hip labrum reconstruction has traditionally been performed by implanting allografts or xenografts made up of natural tissue. However, the such allografts and xenografts may have one or more drawbacks and their mechanical and biological properties cannot be adjusted. WO2015/134028 A1 discloses a degradable medical device prepared from a polyurethane foam. The medical device can be a meniscal implant, a glenoid implant, or a glenoid labrum implant. To promote the growth of tissue, the porous foams preferably have an interconnected porous structure with a diameter of more than 30 µm.

Accordingly, new hip labrum scaffolds are needed.

### SUMMARY

The present disclosure generally provides hip labrum scaffolds.

In some embodiments, a hip labrum scaffold is provided. The hip labrum scaffold comprises a porous foam. The porous foam comprises a synthetic polymer. The porous foam has an average pore size of greater than or equal to 100 microns and less than or equal to 400 microns.

In some embodiments, the porous foam comprises a synthetic polymer. The porous foam has a suture pull-out strength of greater than or equal to 3 N/mm.

In some embodiments, the porous foam comprises a synthetic polymer. The hip labrum scaffold is configured to degrade when positioned in a human patient. The degradation is configured to be completed at a time of greater than or equal to 4 years and less than or equal to 6 years after implantation into the human patient.

Also described, but not claimed, is a method of annealing a porous foam for use in a hip labrum scaffold is provided. The method comprises heating the porous foam to a temperature of at least 1 °C above its melting point, thereby increasing the average pore size of the porous foam. The porous foam comprises a synthetic polymer.

In some embodiments, a method of annealing a porous foam comprises heating the porous foam to a temperature of at least 1 °C above its melting point, thereby increasing one or more mechanical properties of the porous foam while substantially retaining its porosity. The porous foam comprises a synthetic polymer. The one or more mechanical properties are selected from the group consisting of the compressive modulus of the porous foam, a tensile modulus of the porous foam, a tensile strength of the porous foam, and the suture pull-out strength of the porous foam.

In some embodiments, a method of annealing a porous foam comprises heating the porous foam to a temperature of at least 1 °C above its melting point and at most 20 °C above its melting point. The porous foam comprises a synthetic polymer.

Other advantages and novel features of the present invention will become apparent from the following detailed description of various non-limiting embodiments of the invention when considered in conjunction with the accompanying figures.

### BRIEF DESCRIPTION OF THE DRAWINGS

Non-limiting embodiments of the present invention will be described by way of example with reference to the accompanying figures, which are schematic and are not intended to be drawn to scale. In the figures, each identical or nearly identical component illustrated is typically represented by a single numeral. For purposes of clarity, not every component is labeled in every figure, nor is every component of each embodiment of the invention shown where illustration is not necessary to allow those of ordinary skill in the art to understand the invention. In the figures:
FIG. 1 shows one example of a porous foam, in accordance with some embodiments;
FIG. 2 shows another example of a porous foam, in accordance with some embodiments;
FIGs. 3A-3B show schematic depictions of human hips, in accordance with some embodiments;
FIG. 4 shows micrographs of human hip labra, in accordance with some embodiments;
FIGs. 5A-5B show schematic depictions of shapes that hip labrum scaffolds and porous foams may have, in accordance with some embodiments;
FIGs. 6-7 show photographs of a hip labrum scaffold, in accordance with some embodiments;
FIG. 8 shows a schematic depiction of a hip labrum scaffold comprising a triangular groove, in accordance with some embodiments;
FIG. 9 shows a photograph of a hip labrum scaffold, in accordance with some embodiments;
FIGs. 10-11 show schematic depictions of a hip labrum scaffold comprising a groove, in accordance with some embodiments;
FIG. 12 shows a photograph of a hip labrum scaffold, in accordance with some embodiments;
FIG. 13 shows a schematic depiction of a hip labrum scaffold comprising a square groove, in accordance with some embodiments;
FIG. 14 shows a scanning electron micrograph of a porous foam, in accordance with some embodiments;
FIG. 15 shows an x-ray microtomography image of a porous foam, in accordance with some embodiments;
FIG. 16 shows a three-dimensional rendering of a porous foam, in accordance with some embodiments;
FIG. 17 shows a scanning electron micrograph of a porous foam, in accordance with some embodiments;
FIGs. 18-19 show features of the pores of two porous foams, in accordance with some embodiments;
FIG. 20 shows the results of various tests performed on porous foams, in accordance with some embodiments;
FIG. 21 shows the aggregate modulus of a porous foam for various applied compressive strains, in accordance with some embodiments;
FIG. 22 shows the permeability of a porous foam for various applied compressive strains, in accordance with some embodiments;
FIG. 23 shows the time constant of a porous foam for various applied compressive strains, in accordance with some embodiments; and
FIGs. 24-26 show scanning electron microscopy images of a porous foam after the application of various compressive strains.

### DETAILED DESCRIPTION

Hip labrum scaffolds are generally provided. In some embodiments, the hip labrum scaffolds described herein have one or more advantageous features. For instance, some hip labrum scaffolds may have one or more properties that facilitate tissue ingrowth. Desirably, tissue ingrowth may result in the formation of a composite material (e.g., comprising the hip labrum scaffold and ingrown tissue) having mechanical and/or biological properties similar to that of a natural, undamaged hip labrum. Such hip labrum scaffolds, when positioned in a patient, may advantageously cause the patient to experience little pain (e.g., less pain than hip labrum scaffolds having mechanical and/or biological properties dissimilar to those of natural, undamaged hip labrums) and/or have improved functionality (e.g., in comparison to than hip labrum scaffolds having mechanical and/or biological properties dissimilar to those of natural, undamaged hip labrums).

Properties that may promote tissue ingrowth include the presence of pores having one or more properties that promote tissue ingrowth, mechanical properties that promote tissue ingrowth, and/or a chemical composition that promotes tissue ingrowth. For instance, some hip labrum scaffolds may comprise pores in a size, shape, amount, and/or connectivity that facilitate tissue ingrowth. As another example. in some embodiments, a hip labrum scaffold designed herein has a relatively high compressive modulus and/or resistance to pore collapse. Such hip labrum scaffolds may advantageously retain open pores into which tissue grow in a facile manner even when subject to pressures typically applied to the hip labrum during standing and/or locomotion. As a third example, some hip labrum scaffolds may have a chemistry that is nontoxic to cells and/or that allows cell and/or proliferation.

Some hip labrum scaffolds described herein may advantageously be configured to readily seal the acetabulum to the femoral head when implanted in a patient as a hip labrum replacement. The seal may be sufficient to substantially inhibit and/or prevent the flow of synovial fluid out of the sealed cavity bounded by the hip labrum scaffold, the femoral head, and the acetabulum. This may promote the retention of such synovial fluid in this cavity, which may desirably assist with joint lubrication. Some properties that may promote the sealing of the acetabulum to the femoral head include properties that facilitate tissue ingrowth (as tissue ingrowth is believed to assist with such sealing), high flexibility, and high toughness. It is believed that hip labrum scaffolds having relatively high flexibility and/or toughness may be capable of being readily positioned in challenging and/or relatively inaccessible geometries. For instance, they may be reversibly and/or irreversibly deformed to pass through narrow passageways and/or to adapt to geometries in which they are positioned. High flexibility and/or toughness are also believed to assist with preventing failure of the scaffold upon the application of pressures typically applied to the hip labrum during standing and/or locomotion.

The hip labrum scaffolds described herein may have a variety of suitable morphologies. The hip labrum scaffold comprises a plurality of pores. As described elsewhere herein, such pores may promote and/or be configured to promote tissue ingrowth into the hip labrum scaffold. For instance, some hip labrum scaffolds may comprise pores into which tissue and/or one or more tissue components (e.g., cells, extracellular membrane) readily grows.

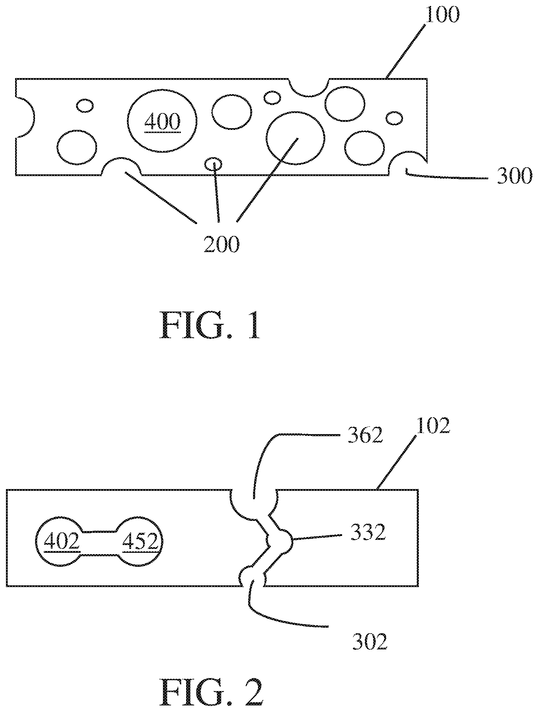

The hip labrum scaffold comprises a porous foam. In other words, the plurality of pores may be positioned in a porous foam. The pores may comprise pores that are filled, pores that are unfilled, or both. If filled, the pores may be filled with one or more gases (e.g., air), one or more liquids (e.g., water, a biological fluid), one or more gels (e.g., one or more biological gels), and/or one or more solids other than the solid enclosing the pores (e.g., one or more solid biological materials). After implantation into a patient, the pores may be filled with tissue and/or cells. FIG. 1 shows a cross-sectional view of one non-limiting embodiment of a porous foam comprising a plurality of pores. In FIG. 1, the porous foam 100 comprises a plurality of pores 200. As shown in FIG. 1, the plurality of pores may comprise both open pores (e.g., the pore 300 shown in FIG. 1) and closed pores (e.g., the pore 400 shown in FIG. 1). When a porous foam comprises both open pores and closed pores, the closed pores may make up a relatively small, or minimal, percentage of the total number and/or volume of pores. It is also possible for a hip labrum scaffold to comprise a porous foam comprising exclusively open pores.

It is also possible for a porous foam to comprise pores that are interconnected with each other. FIG. 2 shows one non-limiting embodiment of a porous foam having this property. In FIG. 2, the porous foam 102 comprises three open pores 302, 332, and 362 that are interconnected. This porous foam also comprises two closed pores 402 and 452 that are interconnected. The porous foams described herein may comprise either, both, or neither of these types of interconnected pores. In some embodiments, like the embodiment shown in FIG. 2, open pores that are interconnected are in fluidic communication with an environment external to the porous foam by exactly two openings. It is also possible for a porous foam to comprise open interconnected pores that are in fluidic communication with an environment external to the porous foam by a number of openings other than two. For instance, in some embodiments, a porous foam comprises open interconnected pores that are in fluidic communication with an environment external to the porous foam by a large number of openings.

Pores that are interconnected may be pores that are in fluidic communication with each other through a pathway that passes exclusively through the interior of the porous foam. In some embodiments, two pores that are interconnected are interconnected such that there are no intervening pores positioned between them. Such pores may be considered to be "directly interconnected" or "in direct fluidic communication" with each other. Unless otherwise specified, pores that are interconnected with each other or in fluidic communication with each other may be directly interconnected or in fluidic communication with each other or may be indirectly (i.e., through one or more pores positioned therebetween) interconnected or in fluidic communication with each other.

The porous foams described herein may have a relatively high porosity and/or comprise pores that have a relatively high degree of interconnectedness. For instance, in some embodiments, a relatively large percentage of the pores may be interconnected with each other through a single network. Such pores may also be in fluidic communication with an environment external to the porous foam by a relatively large number of openings. As an example, a porous foam may comprise a single network through which an appreciable number of pores are interconnected with each other and this network may comprise a relatively large number of openings in the porous foam. It should also be noted that hip labrum scaffolds may comprise porous foams that are open-cell foams and/or porous foams for which the vast majority of pores are open.

When present, the pores of a porous foam may have a variety of suitable sizes. Without wishing to be bound by any particular theory, it is believed that pores having a size in one or more of the ranges described below may facilitate tissue ingrowth to a higher extent than pores of other sizes. Cells are believed to grow in a manner that is influenced by the substrate to which they are anchored. For this reason, it is believed that substrates that have one or more properties similar to an extracellular matrix and/or that cells interact with in a manner similar to an extracellular matrix may facilitate tissue ingrowth better than substrates that differ from extracellular matrices. In particular, it is believed that a porous foam having an average pore size of between 100 microns and 400 microns performs better as an extracellular matrix during tissue ingrowth than porous foams having other pore sizes. Additionally, it is believed that pores that are smaller than 100 microns may prevent tissue ingrowth because they may be too small for cells to infiltrate. It is also believed that pores having a size larger than 400 microns may result in the formation of tissue within the porous foam that has inferior mechanical properties to tissue grown in a porous foam having smaller pores and/or is less homogeneous than tissue grown in a porous foam having smaller pores.

The porous foam comprises pores having an average size of greater than or equal to 100 microns, greater than or equal to 125 microns, greater than or equal to 150 microns, greater than or equal to 175 microns, greater than or equal to 200 microns, greater than or equal to 225 microns, greater than or equal to 250 microns, greater than or equal to 275 microns, greater than or equal to 300 microns, greater than or equal to 325 microns, greater than or equal to 350 microns, or greater than or equal to 375 microns and less than or equal to 400 microns, less than or equal to 375 microns, less than or equal to 350 microns, less than or equal to 325 microns, less than or equal to 300 microns, less than or equal to 275 microns, less than or equal to 250 microns, less than or equal to 225 microns, less than or equal to 200 microns, less than or equal to 175 microns, less than or equal to 150 microns, or less than or equal to 125 microns. Combinations of the above-referenced ranges are also possible (e.g., greater than or equal to 100 microns and less than or equal to 400 microns). Other ranges are also possible. The average pore size may be measured by scanning electron microscopy.

In some embodiments, like the embodiments shown in FIGs. 1 and 2, a porous foam comprises pores having a variety of sizes. In other words, the average pore size may be within one or more of the ranges described in the preceding paragraph, but individual pores may have sizes outside of these ranges. It is also possible for a porous foam to have relatively uniform pores. Similarly, a porous foam may be similar to the porous foam shown in FIGs. 1 and 2 by comprising pores that are relatively spherical and/or have relatively circular cross-sections and/or may differ from the porous foam shown in FIGs. 1 and 2 by having pores having a different shape. For instance, in some embodiments, a porous foam comprises pores that are elongated, have an oval cross-section, are polygonal, and/or have a polygonal cross-section.

In some embodiments, a porous foam comprises both macropores (e.g., pores having an average size in excess of 10 microns, such as pores having an average size of 100 microns or more) and micropores (e.g., pores having an average size of less than 10 microns). In such embodiments, the porous foam may have an average pore size of all of the pores present in the porous foam in one or more of the ranges described above and/or may have an average pore size of all of the pores observable by scanning electron microscopy in one or more of the ranges described above. As described in further elsewhere herein, micropores are believed to be undesirable because they are believed to act as stress concentrators.

Some porous foams advantageously comprise a relatively low amount (or zero) micropores (e.g., pores having an average pore size of less than 10 microns, less than or equal to 8 microns, less than or equal to 6 microns, less than or equal to 4 microns, less than or equal to 2 microns, or less than or equal to 1 micron). For instance, in some embodiments, micropores make up less than or equal to 15 vol%, less than or equal to 12 vol%, less than or equal to 10 vol%, less than or equal to 7.5 vol%, less than or equal to 5 vol%, less than or equal to 2 vol%, less than or equal to 1 vol%, less than or equal to 0.75 vol%, less than or equal to 0.5 vol%, less than or equal to 0.2 vol%, or less than or equal to 0.1 vol% of all of the pores in the porous foam. In some embodiments, micropores make up greater than or equal to 0 vol%, greater than or equal to 0.1 vol%, greater than or equal to 0.2 vol%, greater than or equal to 0.5 vol%, greater than or equal to 0.75 vol%, greater than or equal to 1 vol%, greater than or equal to 2 vol%, greater than or equal to 5 vol%, greater than or equal to 7.5 vol%, greater than or equal to 10 vol%, or greater than or equal to 12 vol% of the pores in the porous foam. Combinations of the above-referenced ranges are also possible (e.g., less than or equal to 15 vol% and greater than or equal to 0 vol%). Other ranges are also possible.

When present, pores may make up a variety of suitable volume fractions of the porous foam. In some embodiments, the porosity of the porous foam (i.e., the volume fraction of the porous foam occupied by pores) is greater than or equal to 70%, greater than or equal to 72.5%, greater than or equal to 75%, greater than or equal to 77.5%, greater than or equal to 80%, greater than or equal to 82.5%, greater than or equal to 85%, or greater than or equal to 87.5%. In some embodiments, the porosity of the porous foam is less than or equal to 90%, less than or equal to 87.5%, less than or equal to 85%, less than or equal to 82.5%, less than or equal to 80%, less than or equal to 77.5%, less than or equal to 75%, or less than or equal to 72.5%. Combinations of the above-referenced ranges are also possible (e.g., greater than or equal to 70% and less than or equal to 90%). Other ranges are also possible. The porosity of a porous foam may be measured in accordance with ASTM F2450-10.

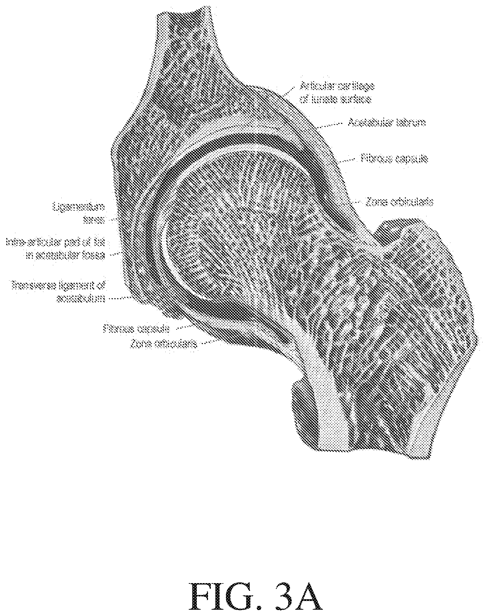

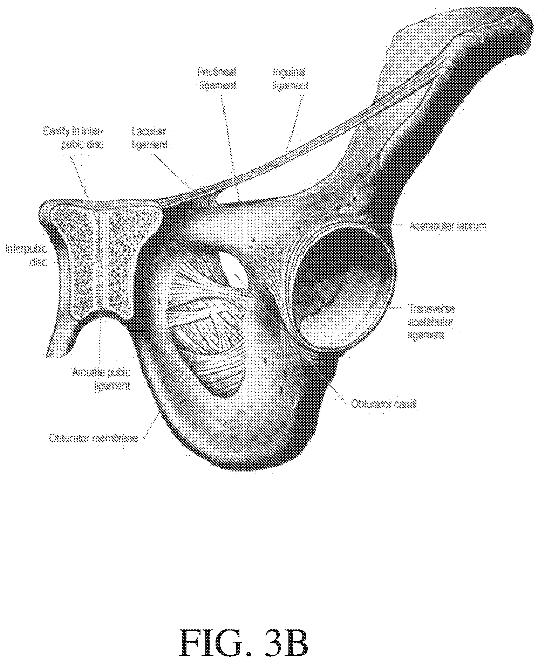

The hip labrum scaffolds described herein may have a variety of suitable shapes and sizes. In some embodiments, a hip labrum scaffold is sized such that, when implanted a patient, it is a suitable replacement for a hip labrum. FIG. 3A is a schematic depiction of a cross-section of a human hip. The hip labrum scaffolds described herein may be configured to be, and/or capable of being, positioned at the location labeled "acetabular labrum" (which is a synonym for hip labrum). As shown in FIG. 3B, the hip labrum extends around the upper surface of the articular cartilage to form a circular rim.

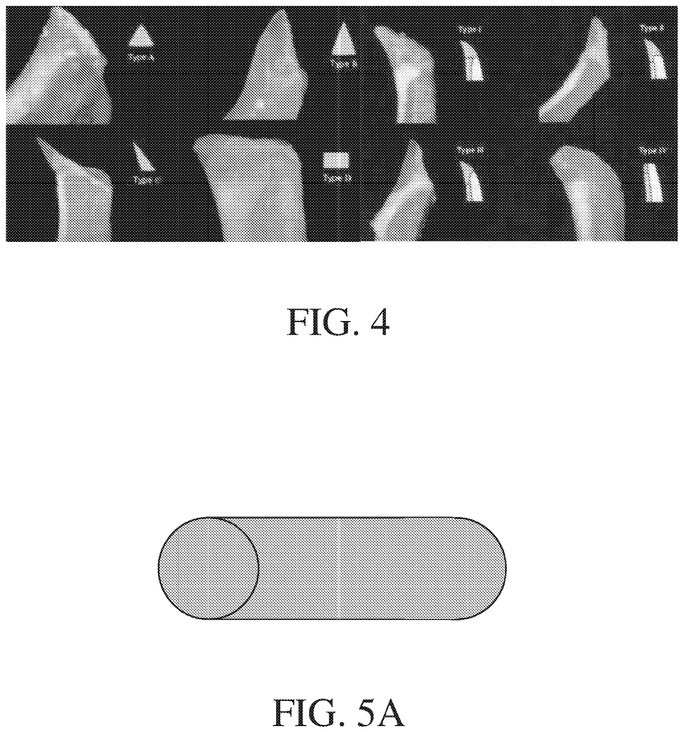

There is natural variability in the size and shape of the cross-section of human hip labrums. Some human hip labrums have triangular cross-sections (e.g., a cross-section like the cross-section shown in FIG. 3A, a cross-section that resembles an equilateral triangle, a cross-section that resembles an isosceles triangle, a cross-section that resembles a scalene triangle, a cross-section that is an acute triangle, a cross-section that is an obtuse triangle, a cross-section that is a right triangle). Some human hip labrums have cross-sections with other shapes. For instance, some human hip labrums have round, semicircular, four-sided, irregular, or flattened cross-sections. Micrographs of human hip labrums having a variety of cross-sections are shown in FIG. 4. These labrums are attached to, and positioned above, articular cartilage. Beside each micrograph in FIG. 4 is a schematic depiction of the relevant cross-section of the hip labrum.

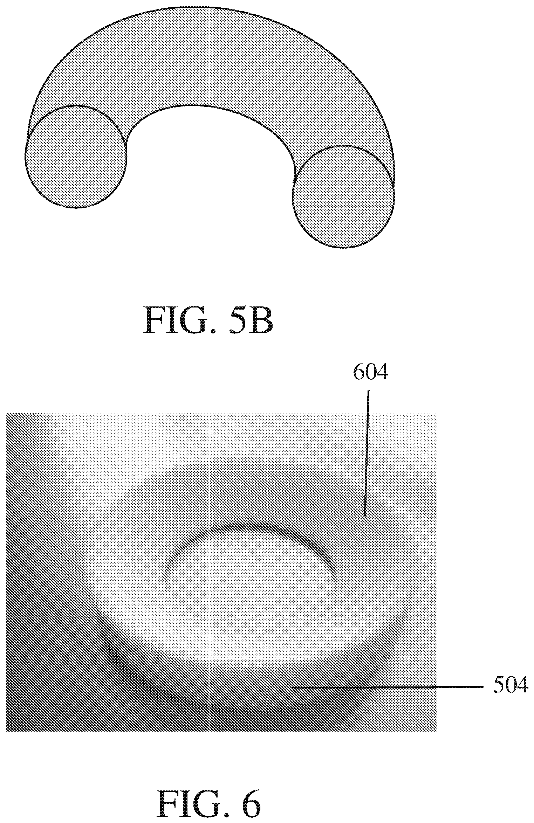

In some embodiments, a hip labrum scaffold, and/or a component thereof (e.g., a porous foam), has a shape that matches the shape of a human hip labrum. For instance, in some embodiments, a hip labrum scaffold and/or a porous foam forms a cylinder or a hollow cylinder having one or more of the cross-sections described above. In other words, a hip labrum scaffold and/or porous foam may have one donut-shaped cross-section (e.g., in a plane perpendicular to the axis of the cylinder) and one cross-section that is triangular, round, semicircular, four-sided, irregular, or flattened (e.g., in a plane containing the axis of the cylinder). When a hip labrum scaffold and/or porous foam forms a cylinder, the cylinder may be straight (e.g., as shown illustratively in FIG. 5A) or curved (e.g., as shown illustratively in FIG. 5B).

It is also possible for a hip labrum scaffold and/or a porous foam to have a shape that is not one found in human patients. Such hip labrum scaffolds may be provided to surgeons, who may then cut the hip labrum scaffold and/or porous foam to a shape that matches the hip labrum of a patient. For instance, some hip labrum scaffolds and/or porous foams may be tubular, ring-shaped, rod-shaped, cigar-shaped, etc. In some embodiments, a hip labrum scaffold and/or a porous foam has a shape that is suitable for some patients but not others (e.g., that has a triangular cross-section). Such hip labrum scaffolds and/or porous foams may also be cut by surgeons to an appropriate shape for patients that have differing-shaped hip labrums than those of the scaffolds.

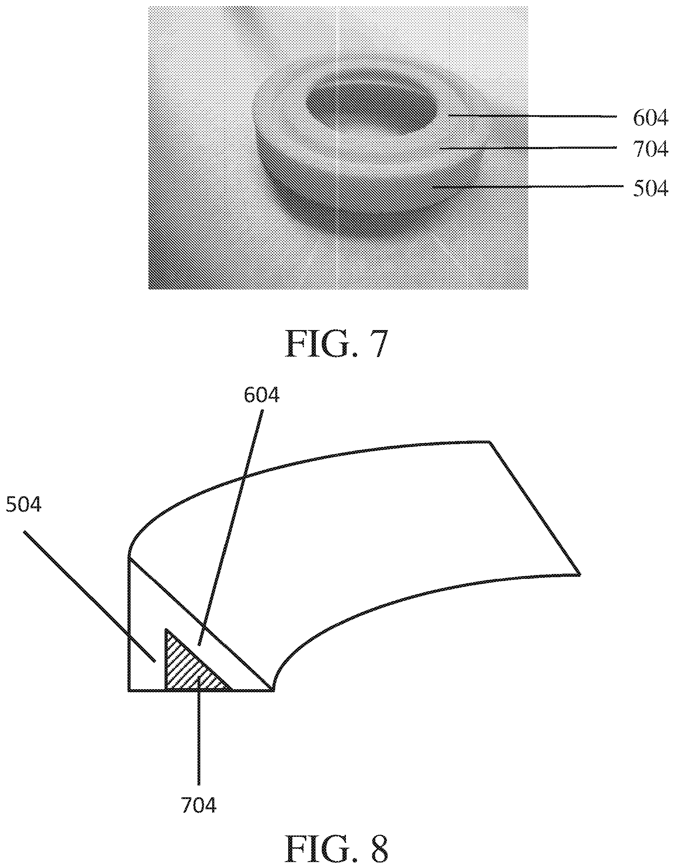

Further examples of shapes that hip labrum scaffolds and/or porous foams may have are shown in FIGs. 6-13. FIGs. 6 and 7 show photographs of the top and bottom of one exemplary hip labrum scaffold. This hip labrum scaffold takes the form of a hollow cylinder where the cylinder wall comprises an exterior portion parallel to the cylindrical axis (labeled 504 in FIGs. 6 and 7) and a portion that extends towards the center of the hip labrum scaffold at an angle from the top of the parallel to portion to the base of the hollow cylinder (labeled 604 in FIGs. 6 and 7). The space between these portions may be a triangular groove (labeled 704 in FIGs. 6 and 7). The groove may be a right triangular groove, where the right angle is at the base of the cylinder. FIG. 8 shows a schematic depiction of this triangular groove, which is shown as the shaded portion. Like in FIGs. 6-8, in some embodiments, a hip labrum scaffold and/or a porous foam comprises a hollow cylinder with one or more sloping side walls and/or one or more grooves. The cross-sections of the grooves (e.g., in a plane perpendicular to the may, in some embodiments, resemble triangles, other polygonal shapes, and/or half-circles. In some embodiments, a hip labrum scaffold and/or porous foam comprises a groove that is positioned in a bottom surface of the hip labrum scaffold and/or porous foam, and/or does not contact any lateral side walls.

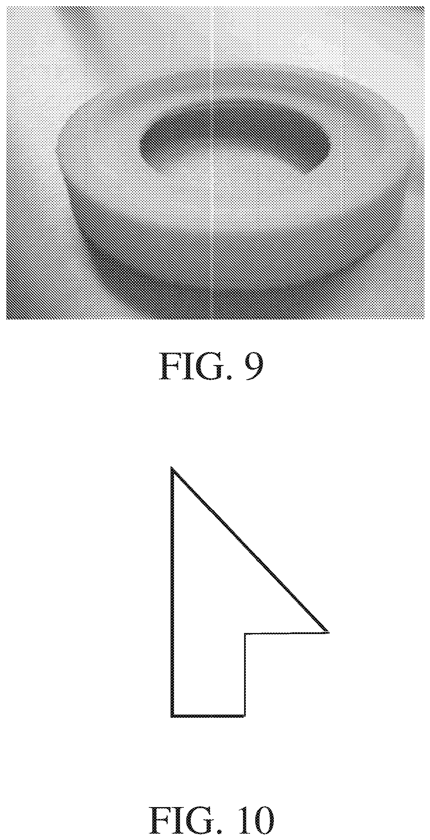

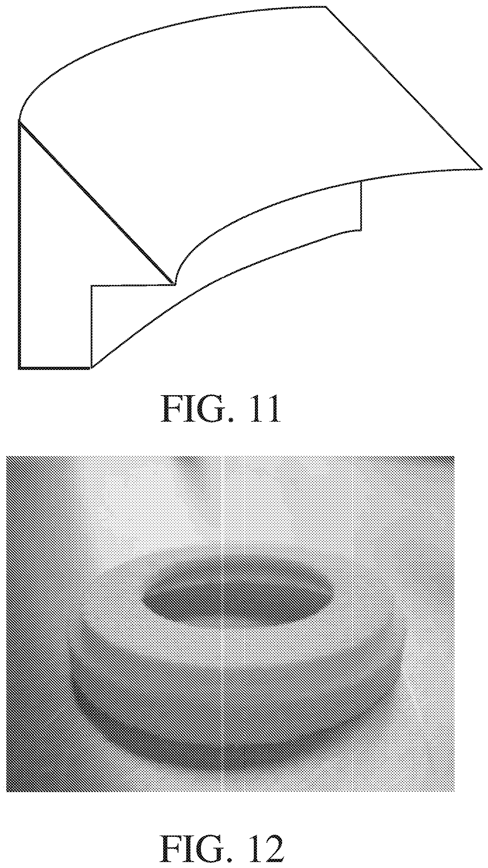

FIG. 9 shows a bottom view of another exemplary hip labrum scaffold. This hip labrum scaffold is similar to that shown in FIGS. 6-7 except that the angled portion has a lower surface that is above the bottom of the hip labrum scaffold. In other words, the hip labrum scaffold shown in FIG. 9 takes the form of a hollow cylinder comprising an inner side wall that is angled towards the center of the hip labrum scaffold. This hip labrum scaffold further comprises a groove that has a polygonal shape and is positioned in both the bottom surface of the hip labrum scaffold and an interior side wall of the cylinder. The inner cylindrical side wall of this hip labrum scaffold is polygonal and comprises a portion that overhangs the groove. These features are further shown schematically in FIGs. 10 and 11.

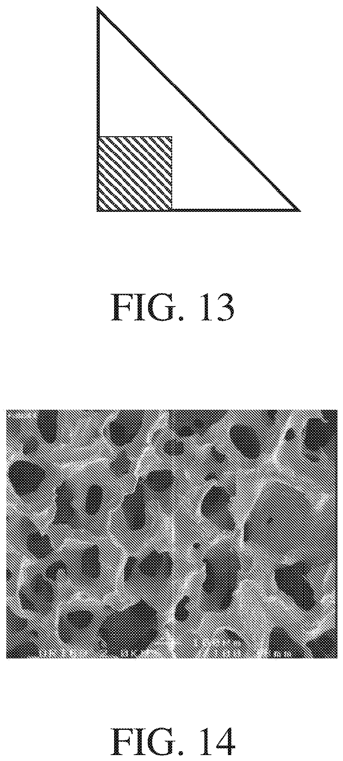

FIG. 12 shows a bottom view of a third exemplary hip labrum scaffold. This hip labrum scaffold takes the form of a hollow cylinder where the cylinder wall is a right triangle from which a rectangle has been removed. In other words, the hip labrum scaffold comprises a rectangular groove that contacts the outer cylinder side wall and the base of the cylinder. The hip labrum scaffold also comprises an inner side wall that is angled towards the center of the hip labrum scaffold. One corner of the rectangle is the right angle in the right triangle. FIG. 13 shows this cross-section schematically.

Hip labrum scaffolds and porous foams may have any suitable maximum width. As used herein, the maximum width of a hip labrum scaffold or a porous foam refers to the longest line segment that may be drawn entirely within the hip labrum scaffold or porous foam and that is perpendicular to the thickness direction of the hip labrum scaffold or porous foam. For hip labrum scaffolds or porous foams taking the form of a hollow cylinder, the thickness direction of the hip labrum scaffold or porous foam is the cylindrical axis. In some embodiments, the maximum width of a hip labrum scaffold and/or porous foam is greater than or equal to 2.5 mm, greater than or equal to 2.75 mm, greater than or equal to 3 mm, greater than or equal to 3.25 mm, greater than or equal to 3.5 mm, greater than or equal to 3.75 mm, greater than or equal to 4 mm, greater than or equal to 4.25 mm, greater than or equal to 4.5 mm, greater than or equal to 4.75 mm, greater than or equal to 5 mm, or greater than or equal to 5.25 mm. In some embodiments, the maximum width of a hip labrum scaffold and/or porous foam is less than or equal to 5.5 mm, less than or equal to 5.25 mm, less than or equal to 5 mm, less than or equal to 4.75 mm, less than or equal to 4.5 mm, less than or equal to 4.25 mm, less than or equal to 4 mm, less than or equal to 3.75 mm, less than or equal to 3.5 mm, less than or equal to 3.25 mm, less than or equal to 3 mm, or less than or equal to 2.75 mm. Combinations of the above-referenced ranges are also possible (e.g., greater than or equal to 2.5 mm and less than or equal to 5.5 mm). Other ranges are also possible.

Hip labrum scaffolds and porous foams may have any suitable maximum thickness. As used herein, the maximum thickness of a hip labrum scaffold or porous foam refers to the longest line segment that may be drawn entirely within the hip labrum scaffold or porous foam and that is parallel to the thickness direction of the hip labrum scaffold or porous foam. In some embodiments, the maximum thickness of a hip labrum scaffold and/or porous foam is greater than or equal to 2.5 mm, greater than or equal to 2.75 mm, greater than or equal to 3 mm, greater than or equal to 3.25 mm, greater than or equal to 3.5 mm, greater than or equal to 3.75 mm, greater than or equal to 4 mm, greater than or equal to 4.25 mm, greater than or equal to 4.5 mm, greater than or equal to 4.75 mm, greater than or equal to 5 mm, greater than or equal to 5.25 mm, greater than or equal to 5.5 mm, greater than or equal to 5.75 mm, greater than or equal to 6 mm, greater than or equal to 6.25 mm, greater than or equal to 6.5 mm, greater than or equal to 6.75 mm, greater than or equal to 7 mm, greater than or equal to 7.25 mm, greater than or equal to 7.5 mm, greater than or equal to 7.75 mm, greater than or equal to 8 mm, or greater than or equal to 8.25 mm. In some embodiments, the maximum thickness of a hip labrum scaffold and/or porous foam is less than or equal to 8.5 mm, less than or equal to 8.25 mm, less than or equal to 8 mm, less than or equal to 7.75 mm, less than or equal to 7.5 mm, less than or equal to 7.25 mm, less than or equal to 7 mm, less than or equal to 6.75 mm, less than or equal to 6.5 mm, less than or equal to 6.25 mm, less than or equal to 6 mm, less than or equal to 5.75 mm, less than or equal to 5.5 mm, less than or equal to 5.25 mm, less than or equal to 5 mm, less than or equal to 4.75 mm, less than or equal to 4.5 mm, less than or equal to 4.25 mm, less than or equal to 4 mm, less than or equal to 3.75 mm, less than or equal to 3.5 mm, less than or equal to 3.25 mm, less than or equal to 3 mm, or less than or equal to 2.75 mm. Combinations of the above-referenced ranges are also possible (e.g., greater than or equal to 2.5 mm and less than or equal to 8.5 mm). Other ranges are also possible.

It should be noted that the hip labrum scaffolds described herein may undergo one or more morphological changes and/or may undergo remodeling after implantation into a patient. For instance, as described in further detail below, in some embodiments, a hip labrum scaffold may degrade over time when present in a patient. As another example, tissue ingrowth may affect the morphology of the hip labrum scaffold over time. Accordingly, in some embodiments, a hip labrum scaffold may independently have a shape and/or size in one or more of the ranges described above prior to implantation in a patient and/or at one or more points in time after implantation into a patient.

As described elsewhere herein, in some embodiments, a hip labrum scaffold and/or porous foam has a chemical composition that is advantageous because it allows tissue ingrowth and/or causes the hip labrum scaffold to have one or more advantageous mechanical properties. Further details regarding possible chemistries are provided below.

Some hip labrum scaffolds and/or porous foams described herein may comprise and/or be formed from synthetic materials, such as synthetic polymers. In some embodiments, a hip labrum scaffold and/or porous foam is exclusively formed from synthetic materials and/or synthetic polymers. In other words, the hip labrum scaffold and/or porous foam may lack biological materials. Such embodiments may be embodiments in which the hip labrum scaffold and/or porous foam has not yet been implanted into a patient (i.e., a hip labrum scaffold and/or porous foam prior to implantation). It is also possible for a hip labrum scaffold and/or a porous foam to comprise biological materials. For instance, such materials may be positioned in at least a portion of the plurality of pores in a hip labrum scaffold and/or porous foam after implantation and at least partial tissue ingrowth. In some such embodiments, the material forming the matrix in which the pores are positioned would lack biological materials.

A variety of suitable types of synthetic polymers may be employed in the hip labrum scaffolds and porous foams described herein. In some embodiments, two or more types of synthetic polymers may be employed in the same hip labrum scaffold and/or porous foam. In such cases, it should be understood that each synthetic polymer may independently have some, all, or none of the features described herein. Of course, it is also possible for a hip labrum scaffold and/or porous foam to be formed from a single type of synthetic polymer. When one or more synthetic polymers are employed in a hip labrum scaffold or synthetic polymer, such polymer(s) may comprise thermoset polymer(s) and/or thermoplastic polymer(s). Similarly, synthetic polymers that are amorphous (e.g., elastomeric), crystalline, and/or semicrystalline may be employed in hip labrum scaffolds and/or porous foams. Homopolymers and copolymers of a variety of types may also be suitable. For instance, in some embodiments, a hip labrum scaffold and/or porous foam comprises a block copolymer. For such polymers, each block may independently be amorphous (e.g., elastomeric), crystalline, and/or semicrystalline.

In some embodiments, a hip labrum scaffold and/or a porous foam comprises a synthetic polymer that is a polyurethane. The polyurethane may comprise hard blocks and soft blocks. The hard blocks may be blocks that are crystalline, semicrystalline, glassy, and/or resistant to deformation. The soft block may be blocks that are rubbery and/or elastomeric. In some embodiments, the polyurethane comprises hard blocks that are phase-separated from the soft blocks. Such hard blocks can act as physical and/or chemical crosslinkers that reduce and/or prevent deformation of the soft blocks beyond a particular distance. Some polyurethanes may comprise polymeric chains that each include a plurality of hard blocks and soft blocks. The hard blocks and soft blocks may be joined together by urethane linkages.

In some embodiments, a species comprising an alcohol group, such as a diol or polyol, reacts with a species comprising an isocyanate group to form the urethane linkage. The diols and polyols may be referred to as "prepolymers", and reactions between such species and species comprising an isocyanate group may be referred to "end-capping". For some diols and polyols, the alcohol groups may be exclusively positioned at the termini of the molecules (i.e., the molecule may have a structure R-(OH)ₙ where n is 2 or greater), may exclusively be positioned in locations other than the termini of the molecules, or may be positioned both at the termini of the molecules and at locations other than the termini of the molecules. Similarly, it is possible for each terminus of a diol or polyol to comprise an alcohol group, for some termini of a diol or polyol to comprise an alcohol group, or for no termini of a diol or polyol to comprise an alcohol group. Non-limiting examples of suitable diols and polyols include small molecule diols and polyols, oligomeric diols and polyols, and macrodiols and macropolyols. The macrodiols and macropolyols may comprise polymers positioned between at least two of the alcohol groups. For diols or polyols, the polymer, oligomer, or small molecule positioned between the alcohol groups in the diol or polyol may be incorporated into the resultant polyurethane as a soft block.

Further examples of suitable diols and polyols (which may be small molecule, oligomeric, or polymeric) include aliphatic diols and polyols (e.g., diols and polyols comprising an aliphatic repeat unit between the alcohol groups, such as a linear aliphatic repeat unit and/or a branched aliphatic repeat unit), cycloaliphatic diols and polyols (e.g., diols and polyols comprising a cycloaliphatic repeat unit between the alcohol groups), diol and polyols comprising a polyester and/or a reaction product of an ester-comprising monomer, diols and polyols comprising a polyether and/or a reaction product of an ether-comprising monomer (e.g., diols and polyols comprising polycaprolactone), diols and polyols comprising a polycarbonate and/or a reaction product of a carbonate-comprising monomer (e.g., diols and polyols comprising 1,6 hexanediol polycarbonate). Non-limiting examples of suitable ester-comprising monomers include ε-caprolactone, lactide, glycolide, and δ-valerolactone. One non-limiting example of a suitable ether-comprising monomers is 1,4-dioxan-2-one. Non-limiting examples of suitable carbonate-comprising monomers include 1,5-dioxepan-2-one, oxepane-2,7-dione, trimethylenecarbonate, tetramethylenecarbonate, 1,3-dioxepan-2-one, and 1,3,8,10-tetraoxacyclotetradecane.

In some examples, a diol or polyol comprises a copolymer. After reaction of the diol or polyol with a species comprising an isocyanate group, the copolymer may be incorporated into a polyurethane as a soft block. The copolymer may comprise one or more of the repeat units described in the preceding paragraph. One example of a suitable copolymer is a copolycarbonate (e.g., that comprises a reaction product of one or more of the carbonate monomers described in the preceding paragraph and comprises a repeat unit that does not comprise a carbonate group).

It is also possible for a diol or polyol to comprise a portion that has a structure other than the structure of a repeat unit in an oligomer or a polymer therein. For instance, in some embodiments, a diol or a polyol is formed by reacting a diol or a polyol with monomers whose polymerization is initiated by the diol or the polyol. In such cases, the species positioned between the alcohol groups may be incorporated into the soft block between polymers or oligomers initiated by the diol or polyol. Non-limiting examples of suitable such diols include C₁-C₁₀ alkyl diols, such as 1,4-butanediol.

Like the diols and polyols described above, species comprising one or more isocyanate groups (e.g., diisocyanates) may also undergo a reaction that results in their reaction products being incorporated into a polyurethane. For such species, the isocyanate groups may be exclusively positioned at the termini of the molecules (i.e., the molecule may have a structure R-(NCO)ₙ where n is 2 or greater), may exclusively be positioned in locations other than the termini of the molecules, or may be positioned both at the termini of the molecules and at locations other than the termini of the molecules. Similarly, it is possible for each terminus of the relevant species to comprise an isocyanate group, for some termini of the relevant species to comprise an isocyanate group, or for no termini of the relevant species to comprise an isocyanate group. Non-limiting examples of suitable species comprising one or more isocyanates include species comprising polymers, oligomers, or small molecules positioned between the isocyanate groups. Also like the diols and polyols described above, the species positioned between the isocyanate groups may be incorporated into the resultant polyurethane. Such species may form hard blocks thereof.

In some embodiments, a species comprising one or more isocyanates comprises (e.g., between the isocyanate groups) a C₂-C₁₄ aliphatic group (e.g., a linear C₂-C₁₄ aliphatic group, a branched C₂-C₁₄ aliphatic group) and/or a cycloaliphatic group. In some embodiments, the C₂-C₁₄ aliphatic group is a C₂-C₁₄ alkylene group (e.g., a linear C₂-C₁₄ alkylene group, a branched C₂-C₁₄ alkylene group). The cycloaliphatic group may be a cycloalkylene group. For any of the above types of C₂-C₁₄ aliphatic groups and/or alkylene groups, the group may be a C₃-C₁₂ group and/or a C₃-C₆ group. Non-limiting examples of suitable diisocyanates include 4,4'-dicyclohexanemethane, 1,4-transcyclohexane-diisocyanate, isophorone diisocyanate, 1,6-hexane diisocyanate, and 1,4-butane diisocyanate.

Some hard blocks may also comprise reaction products of chain extenders. The chain extenders may react with the isocyanate group(s) in the reaction products described above to form longer polymers, a process which may also be referred to as "chain-extending". This process may be performed after the reaction of the species comprising the isocyanate group(s) with the diols and/or polyols. In that first, initial, reaction, the relative amounts of diols, polyols, and species comprising isocyanate groups may be selected such that the majority of the reaction products comprise isocyanate end groups. These isocyanate end groups may further be reacted with the chain extenders to form longer polymers comprising longer hard blocks.

In some embodiments, a chain extender has a structure R-Yₙ (e.g., it has a central R group and one or more Y functional groups that are reactive with isocyanate groups). When n=2, the chain extender has the structure Y-R-Y. For any value of n, each Y may independently by OH (i.e., an alcohol functional group), NH₂ (i.e., a primary amine functional group), or NHR' (i.e., a secondary amine functional group). In some embodiments, all Y are OH (i.e., the chain extender is, itself, a diol or a polyol). It is also possible for all Y to be NH₂ and/or NHR' (i.e., for the chain extender to be a diamine or a polyamine). Y groups that are OH may react with the isocyanate groups to form urethane linkages, and Y groups that are NH₂ or NHR' may react with the isocyanate groups to form urea linkages.

When a chain extender has the structure R-Yₙ, the R and R' groups present may generally be selected as desired. In some embodiments, R is a C₂-C₁₄ aliphatic group (e.g., a linear aliphatic group, a branched aliphatic group, a linear alkylene group, a branched alkylene group) and/or a cycloaliphatic group (e.g., a cycloalkylene group). In some embodiments R' is a C₁-C₁₂ aliphatic group (e.g., a linear C₁-C₁₂ aliphatic group, a branched C₁-C₁₂ aliphatic group). For any of the above types of C₂-C₁₄ aliphatic groups and/or alkylene groups, the group may be a C₃-C₁₂ group and/or a C₃-C₆ group.

Non-limiting specific examples of chain extenders for which each Y is NH₂ and R is a C₁-C₁₂ aliphatic group include ethylene diamines (e.g., 1,2-ethylene diamine), propylene diamines, butylene diamines (e.g., 1,4-butane diamine), hexamethylene diamines (e.g., 1,6-hexamethylene diamine). Non-limiting examples in which Y is NH₂ and R is a C₂-C₁₄ cycloalkylene group include 1,4-isophorone diamine and 1,4-cyclohexane diamine.

Non-limiting specific examples of chain extenders for which each Y is OH and R is an alkylene group include ethylene glycol, diethylene glycol, dipropylene glycol, 1,4-butane diol, 1,6-hexane diol, 1,8-octane diol, neopentyl glycol, 1,12-dodecane diol, cyclohexane dimethanol, and 1,4-cyclohexane diol.

Further examples of chain extender for which each Y is OH include diol block chain extenders. Diol block chain extenders may comprise reaction products of diisocyanates and diols. In some embodiments, the diol blocks may be prepared by reacting a diisocyanate with at least two equivalents of a diol, after which the unreacted excess diol may be removed (e.g., by evaporation, by extraction). Non-limiting examples of suitable diol block chain extenders include the reaction product of 1,4-butane diisocyanate (BDI) with 1,4-butanediol (BDO), the reaction product of BDI with 1,6-hexanediol (HDO), and the reaction product of 1,6-hexanediisocyanate (HDI) with HDO. Such diol blocks may have, respectively, the following structures: BDO-BDI-BDO, HDO-BDI-HDO, and HDO-HDI-HDO. It is also possible for a diol block chain extender to comprise five, seven, or more blocks total. For instance, a diol block chain extender may have the structure (BDO/HDO)-(BDI/HDI-BDO/HDO)ₙ where n is 1, 2, 3, 4 5, 6, 7, 8, 9, or 10. In some embodiments, diol block chain extenders are formed by a method that is catalyst-free.

Blocks within a block copolymer, such as a polyurethane (e.g., hard blocks, soft blocks, blocks comprising polyesters like polycaprolactone, blocks comprising polycarbonates and/or copolycarbonates), may have a variety of suitable molecular weights. In some embodiments, a polymer comprises a block having a number average molecular weight of greater than or equal to 0.6 kg/mol, greater than or equal to 0.7 kg/mol, greater than or equal to 0.8 kg/mol, greater than or equal to 0.9 kg/mol, greater than or equal to 1 kg/mol, greater than or equal to 1.1 kg/mol, greater than or equal to 1.2 kg/mol, greater than or equal to 1.3 kg/mol, greater than or equal to 1.4 kg/mol, greater than or equal to 1.5 kg/mol, greater than or equal to 1.6 kg/mol, greater than or equal to 1.7 kg/mol, greater than or equal to 1.8 kg/mol, greater than or equal to 1.9 kg/mol, greater than or equal to 2 kg/mol, greater than or equal to 2.1 kg/mol, greater than or equal to 2.2 kg/mol, greater than or equal to 2.3 kg/mol, greater than or equal to 2.4 kg/mol, greater than or equal to 2.5 kg/mol, greater than or equal to 2.6 kg/mol, greater than or equal to 2.7 kg/mol, greater than or equal to 2.8 kg/mol, or greater than or equal to 2.9 kg/mol. In some embodiments, a polymer comprises a block having a number average molecular weight of less than or equal to 3 kg/mol, less than or equal to 2.9 kg/mol, less than or equal to 2.8 kg/mol, less than or equal to 2.7 kg/mol, less than or equal to 2.6 kg/mol, less than or equal to 2.5 kg/mol, less than or equal to 2.4 kg/mol, less than or equal to 2.3 kg/mol, less than or equal to 2.2 kg/mol, less than or equal to 2.1 kg/mol, less than or equal to 2 kg/mol, less than or equal to 1.9 kg/mol, less than or equal to 1.8 kg/mol, less than or equal to 1.7 kg/mol, less than or equal to 1.6 kg/mol, less than or equal to 1.5 kg/mol, less than or equal to 1.4 kg/mol, less than or equal to 1.3 kg/mol, less than or equal to 1.2 kg/mol, less than or equal to 1.1 kg/mol, less than or equal to 1 kg/mol, less than or equal to 0.9 kg/mol, less than or equal to 0.8 kg/mol, or less than or equal to 0.7 kg/mol. Combinations of the above-referenced ranges are also possible (e.g., greater than or equal to 0.6 kg/mol and less than or equal to 3 kg/mol, or greater than or equal to 1 kg/mol and less than or equal to 2 kg/mol). Other ranges are also possible.

It should be understood that, when a block copolymer comprises more than one block, each block may independently have a number average molecular weight in one or more of the ranges described above. Similarly, when a polymer comprises multiple blocks of a single type (e.g., multiple soft blocks, multiple hard blocks, multiple blocks comprising polyesters like polycaprolactone, multiple blocks comprising polycarbonates and/or copolycarbonates), the weight average molecular weight averaged across all the blocks of a single type may independently be in one or more of the ranges described above for each type of block.

The number average molecular weight of a block in a block copolymer may be determined by gel permeation chromatography.

A synthetic polymer present in a hip labrum scaffold and/or porous foam may, as a whole, have a variety of suitable number average molecular weights. In some embodiments, a hip labrum scaffold and/or a porous foam comprises a synthetic polymer having a number average molecular weight of greater than or equal to 100 kg/mol, greater than or equal to 105 kg/mol, greater than or equal to 110 kg/mol, greater than or equal to 115 kg/mol, greater than or equal to 120 kg/mol, greater than or equal to 125 kg/mol, greater than or equal to 130 kg/mol, greater than or equal to 135 kg/mol, greater than or equal to 140 kg/mol, greater than or equal to 145 kg/mol, greater than or equal to 150 kg/mol, greater than or equal to 165 kg/mol, greater than or equal to 170 kg/mol, greater than or equal to 175 kg/mol, greater than or equal to 180 kg/mol, greater than or equal to 185 kg/mol, greater than or equal to 190 kg/mol, greater than or equal to 195 kg/mol, greater than or equal to 200 kg/mol, greater than or equal to 205 kg/mol, greater than or equal to 210 kg/mol, greater than or equal to 215 kg/mol, greater than or equal to 220 kg/mol, greater than or equal to 225 kg/mol, greater than or equal to 230 kg/mol, or greater than or equal to 235 kg/mol. In some embodiments, a hip labrum scaffold and/or a porous foam comprises a synthetic polymer having a number average molecular weight of less than or equal to 240 kg/mol, less than or equal to 235 kg/mol, less than or equal to 230 kg/mol, less than or equal to 225 kg/mol, less than or equal to 220 kg/mol, less than or equal to 215 kg/mol, less than or equal to 210 kg/mol, less than or equal to 205 kg/mol, less than or equal to 200 kg/mol, less than or equal to 195 kg/mol, less than or equal to 190 kg/mol, less than or equal to 185 kg/mol, less than or equal to 180 kg/mol, less than or equal to 175 kg/mol, less than or equal to 170 kg/mol, less than or equal to 165 kg/mol, less than or equal to 160 kg/mol, less than or equal to 155 kg/mol, less than or equal to 150 kg/mol, less than or equal to 145 kg/mol, less than or equal to 140 kg/mol, less than or equal to 135 kg/mol, less than or equal to 130 kg/mol, less than or equal to 125 kg/mol, less than or equal to 120 kg/mol, less than or equal to 115 kg/mol, less than or equal to 110 kg/mol, or less than or equal to 105 kg/mol. Combinations of the above-referenced ranges are also possible (e.g., greater than or equal to 100 kg/mol and less than or equal to 240 kg/mol , or greater than or equal to 100 kg/mol and less than or equal to 150 kg/mol). Other ranges are also possible.

When a hip labrum implant and/or a porous foam includes two or more types of synthetic polymers, each synthetic polymer may independently have a number average molecular weight in one or more of the ranges described in the preceding paragraph and/or all of the synthetic polymers together in the hip labrum implant and/or porous foam may have a number average molecular weight in one or more of the ranges described in the preceding paragraph.

The number average molecular weight of a synthetic polymer may be determined by gel permeation chromatography.

A synthetic polymer present in a hip labrum scaffold and/or porous foam may, as a whole, have a variety of polydispersities. In some embodiments, a hip labrum scaffold and/or porous foam has a polydispersity of less than or equal to 3, less than or equal to 2.75, less than or equal to 2.5, less than or equal to 2.25, less than or equal to 2, less than or equal to 1.75, less than or equal to 1.5, or less than or equal to 1.25. In some embodiments, a hip labrum scaffold and/or porous foam has a polydispersity of greater than or equal to 1, greater than or equal to 1.25, greater than or equal to 1.5, greater than or equal to 1.75, greater than or equal to 2, greater than or equal to 2.25, greater than or equal to 2.5, or greater than or equal to 2.75. Combinations of the above-referenced ranges are also possible (e.g., greater than or equal to 1 and less than or equal to 3). Other ranges are also possible.

When a hip labrum implant and/or a porous foam includes two or more types of synthetic polymers, each synthetic polymer may independently have a polydispersity in one or more of the ranges described in the preceding paragraph and/or all of the synthetic polymers together in the hip labrum implant and/or porous foam may have a polydispersity in one or more of the ranges described in the preceding paragraph.

The polydispersity of a synthetic polymer may be determined by gel permeation chromatography.

In some embodiments, a synthetic polymer present in a hip labrum scaffold and/or a porous foam comprises a relatively low level of impurities. For instance, in some embodiments, such a synthetic polymer comprises a relatively low amount of catalyst. The catalyst may be present during synthesis of the synthetic polymer (e.g., it may catalyze the polymerization of monomers to form the synthetic polymer), but then mostly or entirely removed after the conclusion of this synthesis. Non-limiting examples of such catalysts include alkyl tin carboxylates, alkyl tin oxides, alkyl tin mercaptides, triethylenediamine, dimethylcyclohexylamine, dimethylethanolamine, bis-(2-dimethylaminoethyl)ether and dibutyltin dilaurate. It is also possible for a synthetic polymer to be synthesized in a catalyst-free manner.

In some embodiments, less than or equal to 0.001 wt%, less than or equal to 0.0001 wt%, and/or 0 wt% of a hip labrum scaffold, a porous foam, and/or a mixture reacting to form a synthetic polymer is made up of any particular catalyst. It is also possible for all types of catalysts to together make up less than or equal to 0.001 wt%, less than or equal to 0.0001 wt%, and/or 0 wt% of a hip labrum scaffold, a porous foam, and/or a mixture reacting to form a synthetic polymer.

It should be noted that a hip labrum scaffold, porous foam and/or mixture reacting to form a synthetic polymer may comprise any particular catalyst in one or more of the amounts in the preceding paragraph (e.g., in addition to other catalysts). It is also possible for a hip labrum scaffold, porous foam and/or mixture reacting to form a synthetic polymer to comprise a total amount of all catalysts in one or more of the ranges in the preceding paragraph.

A variety of techniques may employed to form synthetic polymers suitable for use in the hip labrum scaffolds and porous foams described herein. For instance, some synthetic polymers may be prepared by step growth techniques and some synthetic polymers may be prepared by chain growth techniques. Non-limiting examples of suitable chain growth techniques include free radical polymerization, cationic polymerization, and anionic polymerization. One specific method suitable for preparing polyurethanes for use in the hip labrum scaffolds and porous foams described herein is described in further detail below.

In some embodiments, synthesizing a polyurethane comprises forming a macrodiol (e.g. a macrodiol having one or more of the chemical compositions described elsewhere herein). The macrodiol may be formed by reacting a diol with a monomer. In some embodiments, the alcohol groups present in the diol initiate ring opening polymerization of the monomer. After which, the monomer polymerizes onto the growing chain at both ends to form a macrodiol. The macrodiol may comprise repeat units that are formed by the polymerized monomer. Such repeat units are described elsewhere herein. The reaction to form a macrodiol may be performed in the presence of a solvent or in the bulk. Non-limiting examples of suitable solvents include dimethylsulfoxide, dimethylformamide, chloroform, 1,4-dioxane, N-methylpyrrolidone, and m-cresol.

Ring opening polymerization may be carried out until a large percentage of the monomers initially present in the reaction mixture are incorporated into the macrodiol. Without wishing to be bound by any particular theory, it is believed that this may reduce the amount of unreacted monomer present in the final product. As it is believed that unreacted monomer present in the final product is often erroneously included in the calculated amount of macrodiol, and the stoichiometry of various reagents in subsequent reactions is often selected based on a calculated amount of macrodiol, reducing the amount of unreacted monomer in the final product may advantageously facilitate better control over the synthesis of the polyurethane. In some embodiments, a macrodiol is formed by performing a reaction until greater than or equal to 99.5 mol% or greater than or equal to 99.8 mol% of the initial monomer content of the reaction mixture is incorporated into the macrodiol. The amount of the initial monomer content incorporated into the macrodiol may be determined by nuclear magnetic resonance.

The temperature of the reaction mixture during the formation of the macrodiol may generally be selected as desired. In some embodiments, the temperature is greater than or equal to 140 °C, greater than or equal to 145 °C, greater than or equal to 150 °C, greater than or equal to 155 °C, greater than or equal to 160 °C, or greater than or equal to 165 °C. In some embodiments, the temperature is less than or equal to 170 °C, less than or equal to 165 °C, less than or equal to 160 °C, less than or equal to 155 °C, less than or equal to 150 °C, or less than or equal to 145 °C. Combinations of the above-referenced ranges are also possible (e.g., greater than or equal to 140 °C and less than or equal to 170 °C). Other ranges are also possible.

After a macrodiol is formed, it may be reacted with a diisocyanate to form a macrodiisocyanate. In other words, the macrodiol may be reacted with two equivalents of a diisocyanate to produce a longer chain capped by isocyanate groups. In the reaction product, the structure initially positioned between the two diol groups in the macrodiol may be joined on both ends by urethane linkages to the structure initially positioned between the isocyanate groups in the diisocyanate. In the reaction mixture, an excess of diisocyanate to macrodiol may be provided. The ratio of diisocyanate to macrodiol may be 2 (e.g., so that, stoichiometrically, the macrodiol would react with the diisocyanate to form a macrodiisocyanate) or may be greater. Without wishing to be bound by any particular theory, it is believed that larger ratios of diisocyanate to macrodiol may impede the formation of species in which two or more macrodiols react with a single diisocyanate to form a longer chain macrodiisocyanate (e.g., a macrodiisocyanate having the structure (isocyanate)-(urethane linkage)-[(species initially positioned between alcohol functional groups in macrodiol)-(urethane linkage)-(species initially positioned between isocyanate functional groups in diisocyanate)-(species initially positioned between alcohol functional groups in macrodiol)]ₙ-(urethane linkage)-(isocyanate) where n is 1 or greater).

In some embodiments, the ratio of diisocyanate to macrodiol is greater than or equal to 2, greater than or equal to 2.5, greater than or equal to 3, greater than or equal to 3.5, greater than or equal to 4, greater than or equal to 4.5, greater than or equal to 5, greater than or equal to 5.5, greater than or equal to 6, greater than or equal to 6.5, greater than or equal to 7, greater than or equal to 7.5, greater than or equal to 8, or greater than or equal to 8.5. In some embodiments, the ratio of diisocyanate to macrodiol is less than or equal to 9, less than or equal to 8.5, less than or equal to 8, less than or equal to 7.5, less than or equal to 7, less than or equal to 6.5, less than or equal to 6, less than or equal to 5.5, less than or equal to 5, less than or equal to 4.5, less than or equal to 4, less than or equal to 3.5, less than or equal to 3, or less than or equal to 2.5. Combinations of the above-referenced ranges are also possible (e.g., greater than or equal to 2 and less than or equal to 9). Other ranges are also possible.

The temperature of the reaction mixture during the formation of the macrodiisocyanate may generally be selected as desired. In some embodiments, the temperature is greater than or equal to 50 °C, greater than or equal to 55 °C, greater than or equal to 60 °C, greater than or equal to 65 °C, greater than or equal to 70 °C, greater than or equal to 75 °C, greater than or equal to 80 °C, greater than or equal to 85 °C, greater than or equal to 90 °C, greater than or equal to 95 °C, greater than or equal to 100 °C, greater than or equal to 105 °C, greater than or equal to 110 °C, or greater than or equal to 115 °C. In some embodiments, the temperature is less than or equal to 120 °C, less than or equal to 115 °C, less than or equal to 110 °C, less than or equal to 105 °C, less than or equal to 100 °C, less than or equal to 95 °C, less than or equal to 90 °C, less than or equal to 85 °C, less than or equal to 80 °C, less than or equal to 75 °C, less than or equal to 70 °C, less than or equal to 65 °C, less than or equal to 60 °C, or less than or equal to 55 °C. Combinations of the above-referenced ranges are also possible (e.g., greater than or equal to 50 °C and less than or equal to 120 °C). Other ranges are also possible.

The time the reaction that the reaction to form the macrodiisocyanate is performed may also generally be selected as desired. In some embodiments, the reaction time is greater than or equal to 3.5 hours, greater than or equal to 4 hours, greater than or equal to 4.5 hours, greater than or equal to 5 hours, greater than or equal to 5.5 hours, greater than or equal to 6 hours, greater than or equal to 6.5 hours, greater than or equal to 7 hours, or greater than or equal to 7.5 hours. In some embodiments, the reaction time is less than or equal to 8 hours, less than or equal to 7.5 hours, less than or equal to 7 hours, less than or equal to 6.5 hours, less than or equal to 6 hours, less than or equal to 5.5 hours, less than or equal to 5 hours, less than or equal to 4.5 hours, or less than or equal to 4 hours. Combinations of the above-referenced ranges are also possible (e.g., greater than or equal to 3.5 hours and less than or equal to 8 hours). Other ranges are also possible.

In some embodiments, a reaction mixture is held at a temperature in one or more of the ranges described above during the entirety of the reaction time. It is also possible for the reaction time to comprise one or more periods of time during which the reaction mixture is held at a temperature in one or more of the ranges described above and period of time during which the reaction mixture is held at a temperature outside of one or more of the ranges described above or for the reaction mixture to be held at temperatures outside of the ranges described above throughout the reaction time.

The reaction to form a macrodiisocyanate may be performed in the presence of a solvent or in the bulk. Non-limiting examples of suitable solvents include dimethylsulfoxide, dimethylformamide, chloroform, 1,4-dioxane, N-methylpyrrolidone, and m-cresol.

After formation of the macrodiisocyanate, unreacted diisocyanate may be removed from the reaction mixture by applying a reduced pressure (e.g., a pressure of less than or equal to 0.01 mbar, a pressure of less than or equal to 0.003 mbar) and/or an elevated temperature thereto (e.g., a temperature between 50 °C and 90 °C). In some embodiments, the reduced pressure and/or the elevated temperature is applied until at least 95 mol% of the initial diisocyanate is incorporated into the macrodiisocyanate or removed, at least 98 mol% of the initial diisocyanate is incorporated into the macrodiisocyanate or removed, at least 99 mol% of the initial diisocyanate is incorporated into the macrodiisocyanate or removed, and/or at least 99.5 mol% of the initial diisocyanate is incorporated into the macrodiisocyanate or removed. The amount of diisocyanate removed may be determined by nuclear magnetic resonance.

After a macrodiisocyanate is formed, it may be reacted with a chain extender. This reaction may be performed in the presence of a solvent or in the bulk. Non-limiting examples of suitable solvents include dimethylsulfoxide, dimethylformamide, chloroform, 1,4-dioxane, N-methylpyrrolidone, and m-cresol. The molar ratio of the chain extender to the macrodiisocyanate in the reaction mixture may be greater than or equal to 1, greater than or equal to 1.01, greater than or equal to 1.02, greater than or equal to 1.03, greater than or equal to 1.04, greater than or equal to 1.05, greater than or equal to 1.06, greater than or equal to 1.07, or greater than or equal to 1.08. The molar ratio of the chain extender to the macrodiisocyanate in the reaction mixture may be less than or equal to 1.09, less than or equal to 1.08, less than or equal to 1.07, less than or equal to 1.06, less than or equal to 1.05, less than or equal to 1.04, less than or equal to 1.03, less than or equal to 1.02, or less than or equal to 1.01. Combinations of the above-referenced ranges are also possible (e.g., greater than or equal to 1 and less than or equal to 1.09). Other ranges are also possible.

Reaction of the macrodiisocyanate with the chain extender may be performed at a variety of suitable temperatures. In some embodiments, the temperature is greater than or equal to 50 °C, greater than or equal to 55 °C, greater than or equal to 60 °C, greater than or equal to 65 °C, greater than or equal to 70 °C, greater than or equal to 75 °C, greater than or equal to 80 °C, greater than or equal to 85 °C, greater than or equal to 90 °C, greater than or equal to 95 °C, greater than or equal to 100 °C, greater than or equal to 105 °C, greater than or equal to 110 °C, greater than or equal to 115 °C, greater than or equal to 120 °C, greater than or equal to 125 °C, greater than or equal to 130 °C, greater than or equal to 135 °C, greater than or equal to 140 °C, or greater than or equal to 145 °C. In some embodiments, the temperature is less than or equal to 150 °C, less than or equal to 145 °C, less than or equal to 140 °C, less than or equal to 135 °C, less than or equal to 130 °C, less than or equal to 125 °C, less than or equal to 120 °C, less than or equal to 115 °C, less than or equal to 110 °C, less than or equal to 105 °C, less than or equal to 100 °C, less than or equal to 95 °C, less than or equal to 90 °C, less than or equal to 85 °C, less than or equal to 80 °C, less than or equal to 75 °C, less than or equal to 70 °C, less than or equal to 65 °C, less than or equal to 60 °C, or less than or equal to 55 °C. Combinations of the above-referenced ranges are also possible (e.g., greater than or equal to 50 °C and less than or equal to 150 °C). Other ranges are also possible.

A variety of techniques may be employed to form a porous foam from a synthetic polymer, such as from a polyurethane. In some embodiments, a method comprises forming a porous scaffold by first forming a mixture in which the synthetic polymer is phase separated from one or more further components, and then removing the further component(s) from the mixture. The locations where the component(s) were positioned prior to removal from the mixture may become pores in the synthetic polymer after removal of the component(s). In one specific example, the mixture comprises a crystalline synthetic polymer, one or more solvents, and a particulate material that is insolvent in the solvent. The synthetic polymer may be miscible with the solvent(s) at higher temperatures and immiscible with the solvent(s) at lower temperatures. Cooling the mixture may thus cause the synthetic polymer to phase microseparate from the solvent(s). If the temperature at which the synthetic polymer and the solvent(s) microphase separate is above the melting point of the synthetic polymer and the melting point of the solvent(s), cooling the mixture may cause liquid-liquid microphase separation between the solvent(s) and the synthetic polymer to occur prior to crystallization of the synthetic polymer or the solvent(s). Then, crystallization of the synthetic polymer and/or solvent(s) upon further cooling may lock the microphase-separated structure in place. After crystallization, the mixture may be washed with a fluid that is a solvent for the particulate material and the solvent(s) but a nonsolvent for the synthetic polymer to remove the particulate material and the solvent(s) from the mixture, leaving behind pores.

It is also possible for a procedure similar to the one described in the preceding paragraph, but in which a pore-forming agent other than a particulate material is employed instead of the particulate material. Non-limiting examples of particulate materials and pore-forming agents include organic small molecules (e.g., sugars, such as saccharose and/or glucose) and salts. The salts may be alkali and/or alkaline salts and/or chloride salts. Non-limiting examples of suitable salts include NaCl, KCl, CaCl₂, and MgCl₂.

When provided as particles, the particle size of a pore-forming agent may generally be selected as desired. In some embodiments, the particulate material has an average diameter of greater than or equal to 30 microns, greater than or equal to 50 microns, greater than or equal to 75 microns, greater than or equal to 100 microns, greater than or equal to 150 microns, greater than or equal to 200 microns, greater than or equal to 250 microns, greater than or equal to 300 microns, greater than or equal to 350 microns, greater than or equal to 400 microns, greater than or equal to 500 microns, greater than or equal to 600 microns, greater than or equal to 700 microns, greater than or equal to 800 microns, greater than or equal to 1 mm, or greater than or equal to 1.25 mm. In some embodiments, the particulate material has an average diameter of less than or equal to 1.5 mm, less than or equal to 1.25 mm, less than or equal to 1 mm, less than or equal to 800 microns, less than or equal to 700 microns, less than or equal to 600 microns, less than or equal to 500 microns, less than or equal to 400 microns, less than or equal to 350 microns, less than or equal to 300 microns, less than or equal to 250 microns, less than or equal to 200 microns, less than or equal to 150 microns, less than or equal to 100 microns, less than or equal to 75 microns, or less than or equal to 50 microns. Combinations of the above-referenced ranges are also possible (e.g., greater than or equal to 30 microns and less than or equal to 1.5 mm). Other ranges are also possible. The average diameter of the particles may be determined by microscopy.

Pore-forming agents may be present in the mixtures described herein in a variety of suitable concentrations. In some embodiments, the pore-forming agent is present in a mixture at a concentration of greater than or equal to 100 wt%/vol, greater than or equal to 125 wt%/vol, greater than or equal to 150 wt%/vol, greater than or equal to 175 wt%/vol, greater than or equal to 200 wt%/vol, greater than or equal to 225 wt%/vol, greater than or equal to 250 wt%/vol, greater than or equal to 275 wt%/vol, greater than or equal to 300 wt%/vol, greater than or equal to 325 wt%/vol, greater than or equal to 350 wt%/vol, or greater than or equal to 375 wt%/vol. In some embodiments, the pore-forming agent is present in the mixture at a concentration of less than or equal to 400 wt%/vol, less than or equal to 375 wt%/vol, less than or equal to 350 wt%/vol, less than or equal to 325 wt%/vol, less than or equal to 300 wt%/vol, less than or equal to 275 wt%/vol, less than or equal to 250 wt%/vol, less than or equal to 225 wt%/vol, less than or equal to 200 wt%/vol, less than or equal to 175 wt%/vol, less than or equal to 150 wt%/vol, or less than or equal to 125 wt%/vol. Combinations of the above-referenced ranges are also possible (e.g., greater than or equal to 100 wt%/vol and less than or equal to 400 wt%/vol). Other ranges are also possible.

In some embodiments, a mixture to which a pore-forming agent, such as a particulate material, is added is heated after such addition. The mixture may be heated to a temperature of greater than or equal to 50 °C, greater than or equal to 60 °C, greater than or equal to 70 °C, greater than or equal to 80 °C, greater than or equal to 90 °C, greater than or equal to 100 °C, greater than or equal to 110 °C, greater than or equal to 120 °C, or greater than or equal to 130 °C. The mixture may be heated to a temperature of less than or equal to 140 °C, less than or equal to 130 °C, less than or equal to 120 °C, less than or equal to 110 °C, less than or equal to 100 °C, less than or equal to 90 °C, less than or equal to 80 °C, less than or equal to 70 °C, less than or equal to 60 °C, or less than or equal to 50 °C. Combinations of the above-referenced ranges are also possible (e.g., greater than or equal to 50 °C and less than or equal to 140 °C). Other ranges are also possible.

Non-limiting examples of suitable solvents include dimethylsulfoxide, dimethyformamide, cresol, 1,4-dioxane, and chloroform. Such solvents may be particularly suitable when the synthetic polymer is a polyurethane.

In some embodiments, a procedure similar to that described above is performed in which the mixture further comprises one or more liquids that are nonsolvents for the synthetic polymer at all temperatures to which the mixture is exposed. The nonsolvent(s) may be added with the solvent and/or may be added to a mixture comprising a synthetic polymer and a solvent. In some embodiments, any nonsolvents are added to such a mixture, and then, subsequently, any pore-forming agents (e.g., particulate materials) are added to the mixture comprising the synthetic polymer, the solvent(s), and the nonsolvent(s). When added to a mixture comprising a synthetic polymer and a solvent, the resultant mixture may then be stirred together. The stirring may take place for a period of time of greater than or equal to 10 minutes, greater than or equal to 12.5 minutes, greater than or equal to 15 minutes, greater than or equal to 17.5 minutes, greater than or equal to 20 minutes, greater than or equal to 22.5 minutes, greater than or equal to 25 minutes, or greater than or equal to 27.5 minutes. The stirring may take place for a period of time of less than or equal to 30 minutes, less than or equal to 27.5 minutes, less than or equal to 25 minutes, less than or equal to 22.5 minutes, less than or equal to 20 minutes, less than or equal to 17.5 minutes, less than or equal to 15 minutes, or less than or equal to 12.5 minutes. Combinations of the above-referenced ranges are also possible (e.g., greater than or equal to 10 minutes and less than or equal to 30 minutes). Other ranges are also possible.

In some embodiments, the mixture comprises a polar nonsolvent, comprises a nonpolar nonsolvent, and/or is miscible with the solvent(s). Non-limiting examples of suitable polar nonsolvents include C₁-C₆ alkyl alcohols (e.g., ethanol) and water. Non-limiting examples of suitable nonpolar nonsolvents include diethyl ether, pentane, and hexane. When the nonsolvent(s) comprise water and the synthetic polymer comprises a polyurethane, it may be desirable to add the water quickly and not stir the mixture for too long. It is believed that the polyurethane may comprise unreacted NCO groups for which the water may serve as a chain extender. It is also believed that it is possible for any unreacted NCO groups to react with the water to form amine groups, which may also be reactive with NCO groups.

In some embodiments, a mixture of a C₁-C₆ alkyl alcohol and water is employed as a nonsolvent. The C₁-C₆ alkyl alcohol may make up greater than or equal to 5 vol%, greater than or equal to 10 vol%, greater than or equal to 15 vol%, greater than or equal to 20 vol%, or greater than or equal to 25 vol% of the alcohol-water mixture. The C₁-C₆ alkyl alcohol may make up less than or equal to 30 vol%, less than or equal to 25 vol%, less than or equal to 20 vol%, less than or equal to 15 vol%, or less than or equal to 10 vol% of the alcohol-water mixture. Combinations of the above-referenced ranges are also possible (e.g., greater than or equal to 5 vol% and less than or equal to 30 vol%). Other ranges are also possible.

When present, the nonsolvent(s) may make up greater than or equal to 2 vol%, greater than or equal to 3 vol%, greater than or equal to 5 vol%, greater than or equal to 7.5 vol%, greater than or equal to 10 vol%, greater than or equal to 12.5 vol%, greater than or equal to 15 vol%, greater than or equal to 17.5 vol%, greater than or equal to 20 vol%, greater than or equal to 22.5 vol%, greater than or equal to 25 vol%, or greater than or equal to 27.5 vol% of the total volume of the solvent(s) and nonsolvent(s) together. In some embodiments, the nonsolvent(s) makes up less than or equal to 30 vol%, less than or equal to 27.5 vol%, less than or equal to 25 vol%, less than or equal to 22.5 vol%, less than or equal to 20 vol%, less than or equal to 17.5 vol%, less than or equal to 15 vol%, less than or equal to 12.5 vol%, less than or equal to 10 vol%, less than or equal to 7.5 vol%, less than or equal to 5 vol%, or less than or equal to 3 vol% of the solvent(s) and the nonsolvent(s) together. Combinations of the above-referenced ranges are also possible (e.g.. greater than or equal to 2 vol% and less than or equal to 30 vol%). Other ranges are also possible.

In some embodiments, a porous foam is prepared according to the procedure described above and the relative amounts of the solvent and the synthetic polymer are selected to enhance the tendency of pores having a desirable morphology to form. For instance, the mixture may comprise relative amounts of the solvent and the synthetic polymer such that, when they form, the pores make up a desirable volume fraction of the mixture. This may be accomplished by causing the volume fraction of the solvent in the phase-separated mixture to match the desired porosity of the porous foam.

In some embodiments, the amount of synthetic polymer is selected such that it is greater than or equal to 40%, greater than or equal to 45%, greater than or equal to 50%, greater than or equal to 55%, greater than or equal to 60%, greater than or equal to 65%, greater than or equal to 70%, greater than or equal to 75%, greater than or equal to 80%, or greater than or equal to 85% of the equilibrium concentration of the synthetic polymer in the synthetic polymer-rich phase of the phase separated mixture at the melting point of the synthetic polymer or solvent. In some embodiments, the amount of synthetic polymer is selected such that it is less than or equal to 90%, less than or equal to 85%, less than or equal to 80%, less than or equal to 75%, less than or equal to 70%, less than or equal to 65%, less than or equal to 60%, less than or equal to 55%, less than or equal to 50%, or less than or equal to 45% of the equilibrium concentration of the synthetic polymer in the synthetic polymer-rich phase of the phase separated mixture at the melting point of the synthetic polymer or solvent. Combinations of the above-referenced ranges are also possible (e.g., greater than or equal to 40% and less than or equal to 90%). Other ranges are also possible. For polyurethanes in polar solvents, in particular, one or more of these ranges may correspond to an amount of polyurethane in the mixture that is between 20 wt%/vol and 50 wt%/vol and/or 30 wt%/vol and 45 wt%/vol.

Without wishing to be bound by any particular theory, it is believed that the volume fraction of each phase in a phase separated mixture may be determined by the lever rule. Using the lever rule, for mixtures consisting of the synthetic polymer and the solvent, the volume fraction of the solvent-rich phase may be determined to be [(volume fraction of the synthetic polymer in the mixture)-(volume fraction of the synthetic polymer in the solvent-rich phase)]/[(volume fraction of the synthetic polymer in the synthetic polymer-rich phase)-(volume fraction of the synthetic polymer in the solvent-rich phase)]. When the volume fraction of the synthetic polymer in the solvent-rich phase is approximately zero, this expression simplifies to (volume fraction of the synthetic polymer in the mixture)/(volume fraction of the synthetic polymer in the synthetic polymer-rich phase).

After formation of a mixture of the synthetic polymer, the solvent(s), the particulate and/or pore-forming agent, and/or the nonsolvent(s), the mixture may be stirred. The stirring may be performed at a variety of suitable temperatures. In some embodiments, the mixture is stirred at a temperature of greater than or equal to 60 °C, greater than or equal to 65 °C, greater than or equal to 70 °C, greater than or equal to 75 °C, greater than or equal to 80 °C, or greater than or equal to 85 °C. In some embodiments, the mixture is stirred at a temperature of less than or equal to 90 °C, less than or equal to 85 °C, less than or equal to 80 °C, less than or equal to 75 °C, less than or equal to 70 °C, or less than or equal to 65 °C. Combinations of the above-referenced ranges are also possible (e.g., greater than or equal to 60 °C and less than or equal to 90 °C). Other ranges are also possible.

The amount of time that stirring is performed may also generally be selected as desired. In some embodiments, the stirring time is greater than or equal to 1 hour, greater than or equal to 1.5 hours, greater than or equal to 2 hours, greater than or equal to 2.5 hours, greater than or equal to 3 hours, greater than or equal to 3.5 hours, greater than or equal to 4 hours, greater than or equal to 4.5 hours, greater than or equal to 5 hours, or greater than or equal to 5.5 hours. In some embodiments, the stirring time is less than or equal to 8 hours, less than or equal to 7.5 hours, less than or equal to 6 hours, less than or equal to 5.5 hours, less than or equal to 5 hours, less than or equal to 4.5 hours, less than or equal to 4 hours, less than or equal to 3.5 hours, less than or equal to 3 hours, less than or equal to 2.5 hours, less than or equal to 2 hours, or less than or equal to 1.5 hours. Combinations of the above-referenced ranges are also possible (e.g., greater than or equal to 1 hour and less than or equal to 6 hours). Other ranges are also possible.

In some embodiments, a mixture is held at a temperature in one or more of the ranges described above during the entirety of the stirring time. It is also possible for the stirring time to comprise one or more periods of time during which the mixture is held at a temperature in one or more of the ranges described above and period of time during which the mixture is held at a temperature outside of one or more of the ranges described above or for the mixture to be held at temperatures outside of the ranges described above throughout the stirring time.

During stirring, the molecular weight of the synthetic polymer may increase.

As described above, in some embodiments, subsequent to the formation of a mixture comprising a synthetic polymer, one or more solvents, one or more pore-forming agents, and/or one or more nonsolvents, the resultant mixture may be placed into a mold and then cooled. The temperature to which the mixture is cooled may be less than or equal to 30 °C, less than or equal to 20 °C, less than or equal to 10 °C, less than or equal to 0 °C, less than or equal to -10 °C, less than or equal to -20 °C, less than or equal to -30 °C, less than or equal to -40 °C, less than or equal to -50 °C, less than or equal to -60 °C, less than or equal to -70 °C, less than or equal to -80 °C, or less than or equal to -90 °C. The temperature to which the mixture is cooled may be greater than or equal to -100 °C, greater than or equal to -90 °C, greater than or equal to -80 °C, greater than or equal to -70 °C, greater than or equal to -60 °C, greater than or equal to -50 °C, greater than or equal to -40 °C, greater than or equal to - 30 °C, greater than or equal to -20 °C, greater than or equal to -10 °C, greater than or equal to 0 °C, greater than or equal to 10 °C, or greater than or equal to 20 °C. Combinations of the above-referenced ranges are also possible (e.g.. less than or equal to 30 °C and greater than or equal to -100 °C). Other ranges are also possible.

Non-limiting examples of fluids suitable for washing the phase-separated, crystallized mixture include organic solvents, aqueous solvents, and mixtures thereof. Non-limiting examples of organic solvents include dimethylsulfoxide, N-methylpyrrolidone, dimethylformamide, dimethyl acetamide, dioxane, and ethanol. Such fluids may be suitable when the synthetic polymer is a polyurethane and/or when the solvent, nonsolvent, and/or particulate materials are those described elsewhere herein. For polyurethanes in particular, it may be desirable to employ the following sequence of fluids for washing the phase-separated, crystallized mixture: a 80 wt%:20 wt% mixture of water:ethanol, a 95 wt%:5 wt% mixture of ethanol:water, and then diethyl ether, hexane, or pentane.

In some embodiments, a further annealing step is performed after the porous foam is washed. The annealing step may comprise heating the porous foam (e.g., at atmospheric pressure) to a temperature above the melting point of the material forming the porous foam (e.g., of the synthetic polymer and/or polymers therein). Without wishing to be bound by any particular theory, it is believed that annealing the porous foam may strengthen it (e.g., it may cause the compressive modulus, tensile modulus, tensile strength, and/or suture pull-out strength of the porous foam to increase) and/or may cause it to develop pores of a size, shape, and/or interconnectivity that are beneficial. This may occur without a substantial decrease in the porosity of the porous foam (e.g., the porosity of the porous foam may be substantially retained while the sizes, shapes, and/or interconnectivities of the pores undergo a change). It is believed that annealing the porous foam may cause smaller pores therein (e.g., having a diameter of less than or equal to 1 micron) to coalesce. It is believed that these smaller pores undesirably act as stress concentrators, and so it is believed that the coalescence of such pores enhances the mechanical properties of the porous foam. It is also believed that the larger pores formed by coalescence promote tissue ingrowth more than the smaller pores undergoing the coalescence, and so such coalescence may also enhance tissue ingrowth into the porous foam.

The temperature to which the porous foam is heated during an annealing step may be greater than or equal to 1 °C, greater than or equal to 2 °C, greater than or equal to 3 °C, greater than or equal to 4 °C, greater than or equal to 5 °C, greater than or equal to 7.5 °C, greater than or equal to 10 °C, greater than or equal to 12.5 °C, greater than or equal to 15 °C, or greater than or equal to 17.5 °C above the melting point of the porous foam. In some embodiments, the temperature to which the porous foam is heated during an annealing step is less than or equal to 20 °C, less than or equal to 17.5 °C, less than or equal to 15 °C, less than or equal to 12.5 °C, less than or equal to 10 °C. less than or equal to 7.5 °C, less than or equal to 5 °C, less than or equal to 4 °C, less than or equal to 3 °C, less than or equal to 2 °C, or less than or equal to 1 °C above the melting point of the porous foam. Combinations of the above-referenced ranges are also possible (e.g.. greater than or equal to 1 °C and less than or equal to 20 °C). Other ranges are also possible. The melting point of the porous foam may be determined by differential scanning calorimetry.

The time for which the annealing step is performed may generally be selected as desired. In some embodiments, the annealing step is performed for a period of time of greater than or equal to 1 minute, greater than or equal to 2 minutes, greater than or equal to 3 minutes, greater than or equal to 4 minutes, greater than or equal to 5 minutes, greater than or equal to 7.5 minutes, greater than or equal to 10 minutes, greater than or equal to 12.5 minutes, greater than or equal to 15 minutes, or greater than or equal to 17.5 minutes. In some embodiments, the annealing step is performed for a period of time of less than or equal to 20 minutes, less than or equal to 17.5 minutes, less than or equal to 15 minutes, less than or equal to 12.5 minutes, less than or equal to 10 minutes, less than or equal to 7.5 minutes, less than or equal to 5 minutes, less than or equal to 4 minutes, less than or equal to 3 minutes, or less than or equal to 2 minutes. Combinations of the above-referenced ranges are also possible (e.g., greater than or equal to 1 minute and less than or equal to 20 minutes). Other ranges are also possible.

In some embodiments, a porous foam is held at a temperature in one or more of the ranges described above during the entirety of the annealing time. It is also possible for the annealing time to comprise one or more periods of time during which the porous foam is held at a temperature in one or more of the ranges described above and period of time during which the porous foam is held at a temperature outside of one or more of the ranges described above or for the porous foam to be held at temperatures outside of the ranges described above throughout the annealing time.

As described elsewhere herein, in some embodiments, a hip labrum scaffold and/or a porous foam has one or more advantageous mechanical properties. Examples of such mechanical properties are described in further detail below.

In some embodiments, a hip labrum scaffold and/or a porous foam has a compressive modulus of greater than or equal to 100 kPa, greater than or equal to 200 kPa, greater than or equal to 300 kPa, greater than or equal to 400 kPa, greater than or equal to 500 kPa, greater than or equal to 600 kPa, greater than or equal to 700 kPa, greater than or equal to 800 kPa, greater than or equal to 900 kPa, greater than or equal to 1000 kPa, greater than or equal to 1100 kPa, greater than or equal to 1200 kPa, greater than or equal to 1300 kPa, or greater than or equal to 1400 kPa. In some embodiments, a hip labrum scaffold and/or a porous foam has a compressive modulus of less than or equal to 1500 kPa, less than or equal to 1400 kPa, less than or equal to 1300 kPa, less than or equal to 1200 kPa, less than or equal to 1100 kPa, less than or equal to 1000 kPa, less than or equal to 900 kPa, less than or equal to 800 kPa, less than or equal to 700 kPa, less than or equal to 600 kPa, less than or equal to 500 kPa, less than or equal to 400 kPa, less than or equal to 300 kPa, or less than or equal to 200 kPa. Combinations of the above-referenced ranges are also possible (e.g., greater than or equal to 100 kPa and less than or equal to 1500 kPa). Other ranges are also possible. The compressive modulus of a hip labrum scaffold or a porous foam may be determined in accordance with ISO 3386/1-1986(E).

In some embodiments, a hip labrum scaffold and/or a porous foam has a tensile strength of greater than or equal to 600 kPa, greater than or equal to 625 kPa, greater than or equal to 650 kPa, greater than or equal to 675 kPa, greater than or equal to 700 kPa, greater than or equal to 725 kPa, greater than or equal to 750 kPa, or greater than or equal to 775 kPa. In some embodiments, a hip labrum scaffold and/or a porous foam has a tensile strength of less than or equal to 800 kPa, less than or equal to 775 kPa, less than or equal to 750 kPa, less than or equal to 725 kPa, less than or equal to 700 kPa, less than or equal to 675 kPa, less than or equal to 650 kPa, or less than or equal to 625 kPa. Combinations of the above-referenced ranges are also possible (e.g., greater than or equal to 600 kPa and less than or equal to 800 kPa). Other ranges are also possible. The tensile strength of a hip labrum scaffold or a porous foam may be determined in accordance with ISO 527-1.

In some embodiments, a hip labrum scaffold and/or a porous foam has a tensile modulus of greater than or equal to 500 kPa, greater than or equal to 525 kPa, greater than or equal to 550 kPa, greater than or equal to 575 kPa, greater than or equal to 600 kPa, greater than or equal to 625 kPa, greater than or equal to 650 kPa, greater than or equal to 675 kPa, greater than or equal to 700 kPa, greater than or equal to 725 kPa, greater than or equal to 750 kPa, or greater than or equal to 775 kPa. In some embodiments, a hip labrum scaffold and/or a porous foam has a tensile modulus of less than or equal to 800 kPa, less than or equal to 775 kPa, less than or equal to 750 kPa, less than or equal to 725 kPa, less than or equal to 700 kPa, less than or equal to 675 kPa, less than or equal to 650 kPa, less than or equal to 625 kPa, less than or equal to 600 kPa, less than or equal to 575 kPa, less than or equal to 550 kPa, or less than or equal to 525 kPa. Combinations of the above-referenced ranges are also possible (e.g., greater than or equal to 500 kPa and less than or equal to 800 kPa). The tensile modulus of a hip labrum scaffold or a porous foam may be determined in accordance with ISO 527-1.

In some embodiments, a hip labrum scaffold and/or a porous foam has a flexibility of greater than or equal to 100%, greater than or equal to 125%, greater than or equal to 150%, greater than or equal to 175%, greater than or equal to 200%, greater than or equal to 225%, greater than or equal to 250%, greater than or equal to 275%, greater than or equal to 300%, greater than or equal to 325%, greater than or equal to 350%, greater than or equal to 375%, greater than or equal to 400%, greater than or equal to 410%, greater than or equal to 420%, greater than or equal to 430%, greater than or equal to 440%, greater than or equal to 450%, greater than or equal to 460%, greater than or equal to 470%, greater than or equal to 480%, or greater than or equal to 490%. In some embodiments, a hip labrum scaffold and/or a porous foam has a flexibility of less than or equal to 500%, less than or equal to 490%, less than or equal to 480%, less than or equal to 470%, less than or equal to 460%, less than or equal to 450%, less than or equal to 440%, less than or equal to 430%, less than or equal to 420%, less than or equal to 410%, less than or equal to 400%, less than or equal to 375%, less than or equal to 350%, less than or equal to 325%, less than or equal to 300%, less than or equal to 275%, less than or equal to 250%, less than or equal to 225%, less than or equal to 200%, less than or equal to 175%, less than or equal to 150%, or less than or equal to 125%. Combinations of the above-referenced ranges are also possible (e.g., greater than or equal to 100% and less than or equal to 500%, or greater than or equal to 400% and less than or equal to 500%). Other ranges are also possible. The flexibility of a hip labrum scaffold or a porous foam may be determined in accordance with ISO 178:2001. The percentages described above refer to the ratio of elongation at break to the distance of the suture from the edge of the sample being tested multiplied by 100%. In some embodiments, the above-referenced flexibility values refer to those measured for samples for which the distance of the suture from the edge of the sample being tested is 3 mm.

In some embodiments, a hip labrum scaffold and/or a porous foam has a suture pull-out strength of greater than or equal to 2 N/mm, greater than or equal to 2.25 N/mm, greater than or equal to 2.5 N/mm, greater than or equal to 2.75 N/mm, greater than or equal to 3 N/mm, greater than or equal to 3.25 N/mm, greater than or equal to 3.5 N/mm, greater than or equal to 3.75 N/mm, greater than or equal to 4 N/mm, greater than or equal to 4.25 N/mm, greater than or equal to 4.5 N/mm, or greater than or equal to 4.75 N/mm. In some embodiments, a hip labrum scaffold and/or a porous foam has a suture pull-out strength of less than or equal to 5 N/mm, less than or equal to 4.75 N/mm, less than or equal to 4.5 N/mm, less than or equal to 4.25 N/mm, less than or equal to 4 N/mm, less than or equal to 3.75 N/mm, less than or equal to 3.5 N/mm, less than or equal to 3.25 N/mm, less than or equal to 3 N/mm, less than or equal to 2.75 N/mm, less than or equal to 2.5 N/mm, or less than or equal to 2.25 N/mm. Combinations of the above-referenced ranges are also possible (e.g., greater than or equal to 2 N/mm and less than or equal to 5 N/mm). Other ranges are also possible.

The suture pull-out strength of a hip labrum scaffold or porous foam may be determined by the following procedure: (1) cutting a hip labrum scaffold or porous foam to form a sample having a thickness of 12 mm; (2) placing two 2-0 MERSILENE braided polyester sutures 3 mm from the edge of sample with a needle; (2) clamping the pointed edge of the sample in one clamp of an Instron tensile tester; (4) clamping both ends of the suture in the other clamp of the Instron tensile tester; and (5) moving the clamps apart at a rate of 10 mm/min. The ratio of the force applied to the hip labrum scaffold or porous foam at failure to the sample thickness is the suture pull-out strength.

In some embodiments, a hip labrum scaffold is configured to, and/or capable of, retaining an appreciable number of pores, an appreciable number of open pores, and/or an appreciable porosity even when subject to an appreciable compressive strain. The retention of the porosity of a hip labrum scaffold after being subject to compressive strain may be determined by: (1) rehydrating a hip labrum scaffold having a diameter of 10 mm and a thickness of between 2 mm and 3 mm overnight in a water bath; (2) placing the rehydrated hip labrum scaffold in a confining chamber having a diameter of 10 mm and a thickness in excess of the thickness of the hip labrum scaffold; (3) filling the remainder of the confining chamber with water; (4) inserting an indenter having a diameter of 10 mm into the confining chamber at a rate of 50 microns per second until a 60% compressive strain is applied; (5) removing the indenter; (6) allowing the hip labrum scaffold to recover for five minutes; (6) compressing the hip labrum scaffold with a porous brass filter having a diameter of 10 mm at a rate of 150 microns per second until 60% compressive strain is applied; (7) applying a constant compressive load to the hip labrum scaffold with the porous brass filter having a magnitude equivalent to the load applied when the 60% compressive strain was initially achieved for three hours; (8) releasing the compressive load; (9) removing the excess water from the hip labrum scaffold; (10) employing a clamp to compress the hip labrum scaffold to the thickness it had at the conclusion of step (7); and (11) imaging the clamped hip labrum scaffold with an SEM.

In some embodiments, when subject to a compressive strain of at least 60%, a hip labrum scaffold retains at least 50%, at least 60%, at least 70%, or at least 80% of the open pores it had prior to application of the compressive strain.

In some embodiments, the hip labrum scaffolds described herein are configured to be implanted into a patient, such as a human patient. Similarly, some embodiments relate to methods of implanting the hip labrum scaffolds described herein into patients (e.g., human patients, patients that are non-human mammals, such as dogs or horses). The hip labrum scaffolds may be configured to be implanted by a variety of suitable techniques, such as arthroscopic techniques, open surgical techniques, and mini-open surgical techniques. For instance, the hip labrum scaffold may be delivered to its target location through a cannula or directly inserted to its target location through an incision. Methods of implanting hip labrum scaffolds may be performed subsequent to the removal of a damaged human hip labrum section from a patient. It is also possible for the implantation of a hip labrum scaffold to occur after a location into which the hip labrum scaffold is to be inserted is prepared for such insertion. As an example, bone anchors may be placed into a patient's acetabular rim to which the hip labrum scaffold may be sutured after implantation.

After the removal of a damaged hip labrum and/or the performance of any other preparatory steps, the hip labrum scaffold may be implanted into the location at which the patient's hip labrum section was positioned prior to removal. For instance, into the location at which the acetabular labrum is shown in FIGs. 3 and 4. In some embodiments, the hip labrum scaffold is implanted, and/or is configured to be implanted, adjacent to a patient's pelvis and/or adjacent to a patient's acetabulum. Once in place, the hip labrum scaffold may be secured, such as with suture anchors and/or sutures. The hip labrum scaffold may be sutured to any bone anchors present and/or to any portions of the acetabular labrum remaining after the hip labrum scaffold has been implanted (e.g., healthy portions that did not need to be removed).

After implantation into a patient, such as a human patient, the hip labrum scaffolds described herein may be configured to degrade. The degradation may be promoted by the chemical environment in which the hip labrum scaffold is positioned (e.g., by the water, salts, and/or other chemicals present in the location in the patient where the hip labrum scaffold is positioned) and/or by one or more biological processes (e.g., by cells). In some embodiments, degradation of a hip labrum scaffold may comprise chemical reactions in which one or more species present in the hip labrum scaffold (e.g., a synthetic polymer) is broken down into two or more smaller, lower molecular weight reaction products. Without wishing to be bound by any particular theory, it is believed that bonds broken by degradation processes may be more susceptible to degradation when they have a higher level of exposure to a surrounding environment. For this reason, it is believed that portions of synthetic polymers having higher levels of contact with biological materials present in a patient may degrade more rapidly than portions of synthetic polymers having lower levels of such contact. As it is also believed that the presence of crystals inhibits contact between bonds positioned in the interior of the crystals and materials in the environment surrounding the crystal, it is believed that crystalline portions of hip labrum scaffolds (e.g., hard blocks in a polyurethane) degrade more slowly than amorphous portions of hip lab scaffolds (e.g., soft blocks and/or elastomeric blocks in a polyurethane).

Additionally, and also without wishing to be bound by any particular theory, it is believed that urethane bonds and ester bonds present in a synthetic polymer may be particularly susceptible to degradation, such as by hydrolysis. For this reason, it is believed that the hip labrum scaffolds described herein comprising these bonds may be prone to degradation that occurs through the degradation of these bonds to produce degradation products (e.g., having lower molecular weights than the material being degraded prior to degradation). It is also believed that ester bonds are more readily degraded in human patients than urethane bonds, and so polyurethanes comprising both types of bonds may degrade through their ester bonds first and then their ether bonds. In some embodiments, the degradation products are non-toxic. For instance, the degradation products may cause minimal or no immune response, sensitization, irritation, cytotoxicity, and/or genotoxicity when present in a human patient at concentrations that would occur upon degradation of the hip labrum scaffold.

Some hip labrum scaffolds that are configured to degrade may be configured to do so relatively slowly. For instance, in some embodiments, a hip labrum scaffold is configured to degrade in a patient (e.g., a human patient) over a period of time of greater than or equal to 4 years, greater than or equal to 4.25 years, greater than or equal to 4.5 years, greater than or equal to 4.75 years, greater than or equal to 5 years, greater than or equal to 5.25 years, greater than or equal to 5.5 years, or greater than or equal to 5.75 years. In some embodiments, a hip labrum scaffold is configured to degrade in a patient over a period of time of less than or equal to 6 years, less than or equal to 5.75 years, less than or equal to 5.5 years, less than or equal to 5.25 years, less than or equal to 5 years, less than or equal to 4.75 years, less than or equal to 4.5 years, or less than or equal to 4.25 years. Combinations of the above-referenced ranges are also possible (e.g.. greater than or equal to 4 years and less than or equal to 6 years). Other ranges are also possible.

The degradation of a hip labrum scaffold may be determined by assessing the molecular weight and chemical composition of the hip labrum scaffold. Full degradation may be determined to have occurred when any remaining components of the hip labrum scaffold have a number average molecular weight of less than or equal to 5% of their initial molecular weight.

For convenience, certain terms employed in the specification, examples, and appended claims are listed here. Definitions of specific functional groups and chemical terms are described in more detail below. For purposes of this invention, the chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Organic Chemistry, Thomas Sorrell, University Science Books, Sausalito: 1999.

The term "aliphatic," as used herein, includes both saturated and unsaturated, nonaromatic, straight chain (i.e., unbranched), branched, acyclic, and cyclic (i.e., carbocyclic) hydrocarbons, which are optionally substituted with one or more functional groups. As will be appreciated by one of ordinary skill in the art, "aliphatic" is intended herein to include, but is not limited to, alkyl, alkenyl, alkynyl, cycloalkyl, cycloalkenyl, and cycloalkynyl moieties. Thus, as used herein, the term "alkyl" includes straight, branched and cyclic alkyl groups. An analogous convention applies to other generic terms such as "alkenyl", "alkynyl", and the like. Furthermore, as used herein, the terms "alkyl", "alkenyl", "alkynyl", and the like encompass both substituted and unsubstituted groups. In certain embodiments, as used herein, "aliphatic" is used to indicate those aliphatic groups (cyclic, acyclic, substituted, unsubstituted, branched or unbranched) having 1-20 carbon atoms. Aliphatic group substituents include, but are not limited to, any of the substituents described herein, that result in the formation of a stable moiety (e.g., aliphatic, alkyl, alkenyl, alkynyl, heteroaliphatic, heterocyclic, aryl, heteroaryl, acyl, oxo, imino, thiooxo, cyano, isocyano, amino, azido, nitro, hydroxyl, thiol, halo, aliphaticamino, heteroaliphaticamino, alkylamino, heteroalkylamino, arylamino, heteroarylamino, alkylaryl, arylalkyl, aliphaticoxy, heteroaliphaticoxy, alkyloxy, heteroalkyloxy, aryloxy, heteroaryloxy, aliphaticthioxy, heteroaliphaticthioxy, alkylthioxy, heteroalkylthioxy, arylthioxy, heteroarylthioxy, acyloxy, and the like, each of which may or may not be further substituted).

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. The alkyl groups may be optionally substituted, as described more fully below. Examples of alkyl groups include, but are not limited to, methyl, ethyl, propyl, isopropyl, butyl, isobutyl, tert-butyl, 2-ethylhexyl, cyclopropyl, cyclobutyl, cyclopentyl, cyclohexyl, and the like. "Heteroalkyl" groups are alkyl groups wherein at least one atom is a heteroatom (e.g., oxygen, sulfur, nitrogen, phosphorus, etc.), with the remainder of the atoms being carbon atoms. Examples of heteroalkyl groups include, but are not limited to, alkoxy, poly(ethylene glycol)-, alkylsubstituted amino, tetrahydrofuranyl, piperidinyl, morpholinyl, etc.

The terms "alkenyl" and "alkynyl" refer to unsaturated aliphatic groups analogous to the alkyl groups described above, but containing at least one double or triple bond respectively. The "heteroalkenyl" and "heteroalkynyl" refer to alkenyl and alkynyl groups as described herein in which one or more atoms is a heteroatom (e.g., oxygen, nitrogen, sulfur, and the like).

The term "aryl" refers to an aromatic carbocyclic group having a single ring (e.g., phenyl), multiple rings (e.g., biphenyl), or multiple fused rings in which at least one is aromatic (e.g., 1,2,3,4-tetrahydronaphthyl, naphthyl, anthryl, or phenanthryl), all optionally substituted. "Heteroaryl" groups are aryl groups wherein at least one ring atom in the aromatic ring is a heteroatom, with the remainder of the ring atoms being carbon atoms. Examples of heteroaryl groups include furanyl, thienyl, pyridyl, pyrrolyl, N lower alkyl pyrrolyl, pyridyl N oxide, pyrimidyl, pyrazinyl, imidazolyl, indolyl and the like, all optionally substituted.

The terms "amine" and "amino" refer to both unsubstituted and substituted amines, e.g., a moiety that can be represented by the general formula: N(R')(R")(R‴) wherein R', R", and R‴ each independently represent a group permitted by the rules of valence.

The terms "acyl," "carboxyl group," or "carbonyl group" are recognized in the art and can include such moieties as can be represented by the general formula: 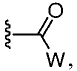 wherein W is H, OH, O-alkyl, O-alkenyl, or a salt thereof. Where W is O-alkyl, the formula represents an "ester." Where W is OH, the formula represents a "carboxylic acid." In general, where the oxygen atom of the above formula is replaced by sulfur, the formula represents a "thiolcarbonyl" group. Where W is a S-alkyl, the formula represents a "thiolester." Where W is SH, the formula represents a "thiolcarboxylic acid." On the other hand, where W is alkyl, the above formula represents a "ketone" group. Where W is hydrogen, the above formula represents an "aldehyde" group.

As used herein, the term "heteroaromatic" or "heteroaryl" means a monocyclic or polycyclic heteroaromatic ring (or radical thereof) comprising carbon atom ring members and one or more heteroatom ring members (such as, for example, oxygen, sulfur or nitrogen). Typically, the heteroaromatic ring has from 5 to about 14 ring members in which at least 1 ring member is a heteroatom selected from oxygen, sulfur, and nitrogen. In another embodiment, the heteroaromatic ring is a 5 or 6 membered ring and may contain from 1 to about 4 heteroatoms. In another embodiment, the heteroaromatic ring system has a 7 to 14 ring members and may contain from 1 to about 7 heteroatoms. Representative heteroaryls include pyridyl, furyl, thienyl, pyrrolyl, oxazolyl, imidazolyl, indolizinyl, thiazolyl, isoxazolyl, pyrazolyl, isothiazolyl, pyridazinyl, pyrimidinyl, pyrazinyl, triazinyl, triazolyl, pyridinyl, thiadiazolyl, pyrazinyl, quinolyl, isoquinolyl, indazolyl, benzoxazolyl, benzofuryl, benzothiazolyl, indolizinyl, imidazopyridinyl, isothiazolyl, tetrazolyl, benzimidazolyl, benzoxazolyl, benzothiazolyl, benzothiadiazolyl, benzoxadiazolyl, carbazolyl, indolyl, tetrahydroindolyl, azaindolyl, imidazopyridyl, qunizaolinyl, purinyl, pyrrolo[2,3]pyrimidyl, pyrazolo[3,4]pyrimidyl, benzo(b)thienyl, and the like. These heteroaryl groups may be optionally substituted with one or more substituents.

The term "substituted" is contemplated to include all permissible substituents of organic compounds, "permissible" being in the context of the chemical rules of valence known to those of ordinary skill in the art. In some cases, "substituted" may generally refer to replacement of a hydrogen with a substituent as described herein. However, "substituted," as used herein, does not encompass replacement and/or alteration of a key functional group by which a molecule is identified, e.g., such that the "substituted" functional group becomes, through substitution, a different functional group. For example, a "substituted phenyl" must still comprise the phenyl moiety and cannot be modified by substitution, in this definition, to become, e.g., a heteroaryl group such as pyridine. In a broad aspect, the permissible substituents include acyclic and cyclic, branched and unbranched, carbocyclic and heterocyclic, aromatic and nonaromatic substituents of organic compounds. Illustrative substituents include, for example, those described herein. The permissible substituents can be one or more and the same or different for appropriate organic compounds. For purposes of this invention, the heteroatoms such as nitrogen may have hydrogen substituents and/or any permissible substituents of organic compounds described herein which satisfy the valencies of the heteroatoms. This invention is not intended to be limited in any manner by the permissible substituents of organic compounds.

Examples of substituents include, but are not limited to, alkyl, aryl, aralkyl, cyclic alkyl, heterocycloalkyl, hydroxy, alkoxy, aryloxy, perhaloalkoxy, aralkoxy, heteroaryl, heteroaryloxy, heteroarylalkyl, heteroaralkoxy, azido, amino, halogen, alkylthio, oxo, acyl, acylalkyl, carboxy esters, carboxyl, carboxamido, nitro, acyloxy, aminoalkyl, alkylaminoaryl, alkylaryl, alkylaminoalkyl, alkoxyaryl, arylamino, aralkylamino, alkylsulfonyl, carboxamidoalkylaryl, carboxamidoaryl, hydroxyalkyl, haloalkyl, alkylaminoalkylcarboxy, aminocarboxamidoalkyl, alkoxyalkyl, perhaloalkyl, arylalkyloxyalkyl, and the like.

When a range of values is listed, it is intended to encompass each value and sub-range within the range. For example "C₁-C₆ alkyl" is intended to encompass, C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆ alkyl.

### EXAMPLE 1

This Example describes the preparation of porous foams comprising a synthetic polymer and the measurement of some of its physical properties. Such porous foams may be suitable for use in hip labrum scaffolds if fabricated to have an appropriate shape.

The samples were initially prepared as described in US 7,943,678. Briefly, the process comprised: (1) synthesizing the poly(ε-caprolactone) (PCL) prepolymer through an uncatalyzed ring opening addition polymerization of ε-caprolactone by employing 1,4-butane diol (BDO) as an initiator; (2) end-capping the resultant PCL prepolymer with butane diisocyanate (BDI); (3) chain-extending the BDI-end-capped PCL with BDO to form the polyurethane; (4) dissolving the resulting polyurethane in dimethylsulfoxide; (5) adding a small amount of water to the solution decrease the solvent quality; (6) adding NaCl particles to the solution to form a slurry; (7) placing the slurry in molds; (8) freezing the molds and the slurries to induce liquid-liquid phase separation; and (9) washing out the NaCl with water and ethanol. Then, the resultant porous foam was annealed for 7 minutes at 108 °C. This annealing treatment enhanced the strength and toughness of the porous foam and while only causing a minimal decrease in its porosity. It also caused the porous foam to shrink slightly.

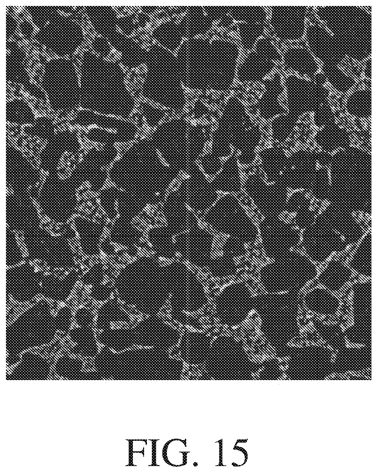

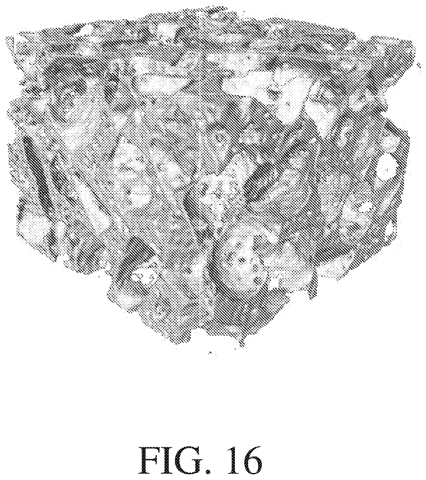

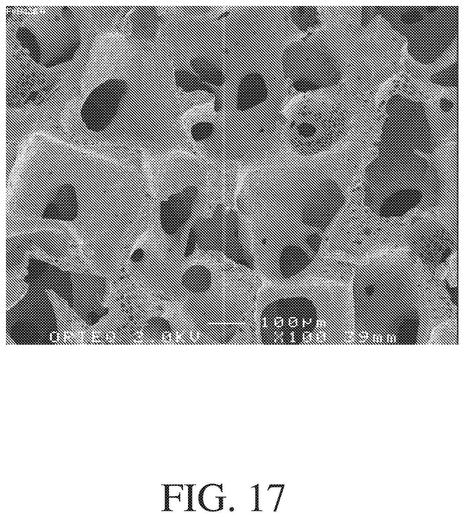

FIG. 14 shows a scanning electron micrograph of the final porous foam, FIG. 15 shows an x-ray microtomography image of the final porous foam, and FIG. 16 shows a three-dimensional rendering of the final porous foam. FIG. 17 shows an electron micrograph of an otherwise equivalent porous foam that did not undergo the annealing process. Table 1 shows several structural and mechanical features of the final porous foam and of the otherwise equivalent porous foam that did not undergo the annealing process.

**Table 1.**

| Property | Annealed Porous Foam | Unannnealed Porous Foam |
|---|---|---|
| Density | 0.3 g/cm³ | 0.2 g/cm³ |
| Suture Pull-Out Strength | 9 N/mm | 4 N/mm |
| Compressive Modulus | 900 kPa | 400 kPa |
| Average Pore Size - Macropores | 100-400 microns | 100-400 microns |
| Pore Morphology | Interconnected | Interconnected |

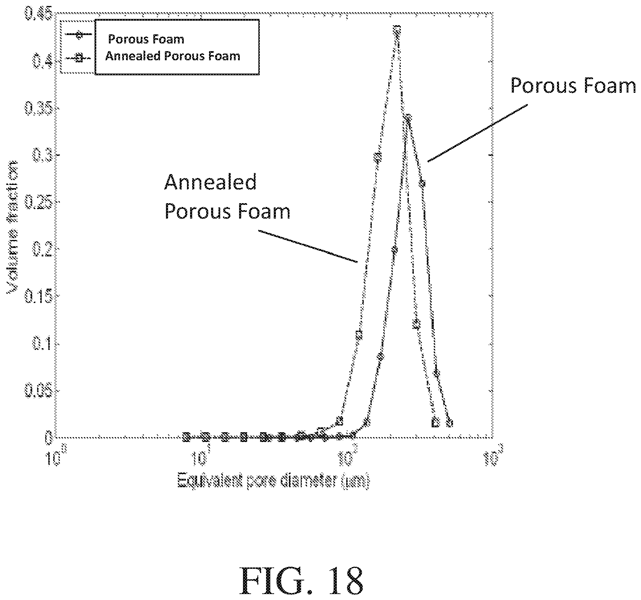

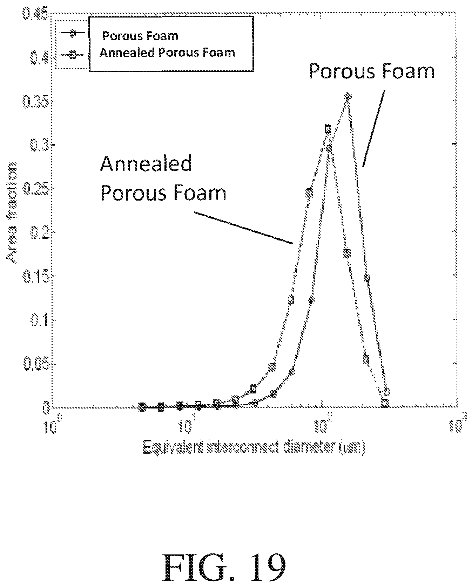

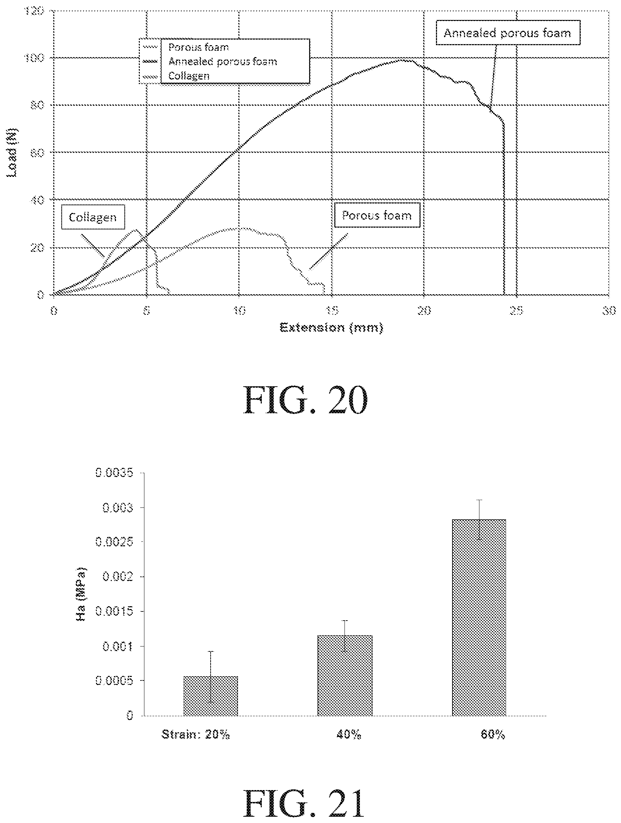

FIGs. 18-19 show features of the pores of the final porous foam in comparison to a porous foam made by a different method. FIG. 20 shows the results of a tensile test performed on the final porous polymer foam to assess its tear strength, the results of an identical tensile test performed on the otherwise equivalent porous foam that did not undergo the annealing process, and the results of an identical tensile test performed on a porous foam formed from collagen.

### EXAMPLE 2

This Example describes the measurement of some of mechanical properties of a porous foam comprising a synthetic polymer. Such porous foams may be suitable for use in hip labrum scaffolds if fabricated to have an appropriate shape.

The porous foam was fabricated by the same procedure described in Example 1. The various properties described below were assessed by employing the same method described above with respect to assessing the open pore retention upon application of a compressive strain and then performing further analysis of the data gathered during that process and/or further testing. However, for some of the mechanical properties measured, values of compressive strain other than 60% were applied, and are indicated as such.

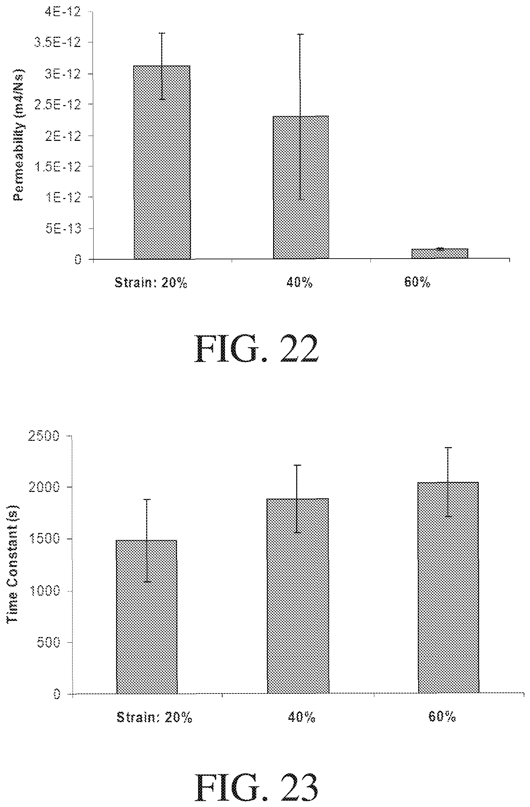

FIG. 21 shows the aggregate modulus for various applied compressive strains, FIG. 22 shows the permeability for various applied compressive strains, and FIG. 23 shows the time constant for various applied compressive strains. Each of these mechanical properties were determined by computationally fitting a mechanical model to the displacement of the indenter over time at each applied strain.

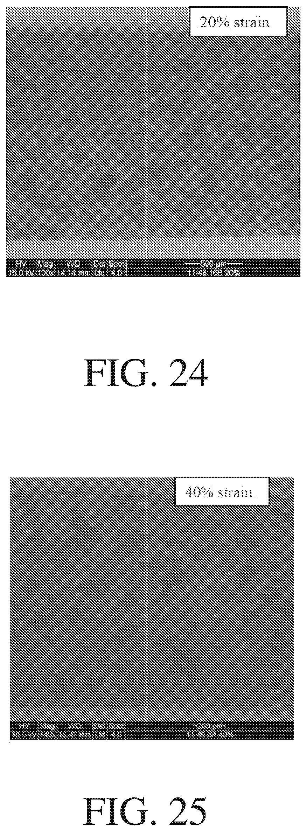

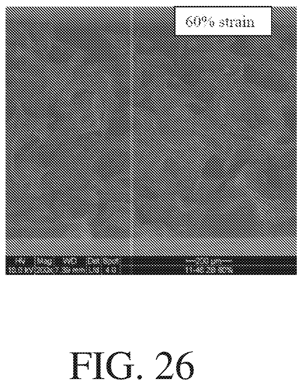

FIGs. 24-26 show scanning electron microscopy images of the porous foam after the application of various compressive strains. In these images, it can be seen that an appreciable number of pores in the porous foam remain open after the application of compressive strain for all three values of compressive strain applied.

While several embodiments of the present invention have been described and illustrated herein, those of ordinary skill in the art will readily envision a variety of other means and/or structures for performing the functions and/or obtaining the results and/or one or more of the advantages described herein, and each of such variations and/or modifications is deemed to be within the scope of the present invention. More generally, those skilled in the art will readily appreciate that all parameters, dimensions, materials, and configurations described herein are meant to be exemplary and that the actual parameters, dimensions, materials, and/or configurations will depend upon the specific application or applications for which the teachings of the present invention is/are used. Those skilled in the art will recognize, or be able to ascertain using no more than routine experimentation, many equivalents to the specific embodiments of the invention described herein. It is, therefore, to be understood that the foregoing embodiments are presented by way of example only and that, within the scope of the appended claims and equivalents thereto, the invention may be practiced otherwise than as specifically described and claimed. The present invention is directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the scope of the present invention.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified. Thus, as a non-limiting example, a reference to "A and/or B", when used in conjunction with open-ended language such as "comprising" can refer, in one embodiment, to A only (optionally including elements other than B); in another embodiment, to B only (optionally including elements other than A); in yet another embodiment, to both A and B (optionally including other elements); etc.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of." "Consisting essentially of," when used in the claims, shall have its ordinary meaning as used in the field of patent law.

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified. Thus, as a non-limiting example, "at least one of A and B" (or, equivalently, "at least one of A or B," or, equivalently "at least one of A and/or B") can refer, in one embodiment, to at least one, optionally including more than one, A, with no B present (and optionally including elements other than B); in another embodiment, to at least one, optionally including more than one, B, with no A present (and optionally including elements other than A); in yet another embodiment, to at least one, optionally including more than one, A, and at least one, optionally including more than one, B (and optionally including other elements); etc.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of' shall be closed or semi-closed transitional phrases, respectively, as set forth in the United States Patent Office Manual of Patent Examining Procedures, Section 2111.03.

## Claims

1. A hip labrum scaffold, comprising:
a porous foam, wherein:
the porous foam comprises a synthetic polymer; and
the porous foam has an average pore size of greater than or equal to 100 microns and less than or equal to 400 microns,
wherein the hip labrum scaffold is sized such that, when implanted in a patient, it is a suitable replacement for the hip labrum.

2. The hip labrum scaffold of claim 1, wherein the porous foam is annealed, wherein the annealing is optionally achieved by
heating the porous foam to a temperature of at least 1 °C above its melting point, thereby increasing the average pore size of the porous foam.

3. The hip labrum scaffold of claim 1 or 2, wherein the polymer is a polyurethane, and, optionally, wherein;
(a) the polyurethane comprises an aliphatic repeat unit, optionally, wherein:
(i) the aliphatic repeat unit is linear; or
(ii) the aliphatic repeat unit is branched; and/or
(b) the polyurethane comprises a cycloaliphatic repeat unit.

4. The hip labrum scaffold as in any preceding claim, wherein the polymer:
(a) comprises a polycaprolactone block, wherein optionally, the polycaprolactone block has a molecular weight of greater than or equal to 1 kDa and less than or equal to 2 kDa; and/or
(b) comprises a reaction product of a macrodiol, optionally, wherein:
(i) the macrodiol comprises a reaction product of ε-caprolactone, lactide, glycolide, δ-valerolactone, 1,4-dioxane-2-one, 1,5-dioxepan-2-one, oxepan-2,7-dione, a polycarbonate, and/or a copolycarbonate, optionally, wherein the copolycarbonate comprises a reaction product of trimethylenecarbonate, tetramethylenecarbonate, 1,3-dioxepan-2-one, and/or 1,3,8,10-tetraoxacyclotetradecane; and/or
(ii) the macrodiol comprises 1,6-hexanediol polycarbonate; and/or
(iii) the macrodiol comprises a reaction product of a C₁-C₁₀ alkyl diol, optionally, wherein the C₁-C₁₀ alkyl diol is 1,4-butanediol; and/or
(c) has a number average molecular weight of greater than or equal to 100 kg/mol and less than or equal to 150 kg/mol; and/or
(d) is semicrystalline; and/or
(e) comprises an elastomeric block.

5. The hip labrum scaffold as in any preceding claim, wherein the polymer comprises a reaction product of a diisocyanate, optionally, wherein:
(a) the diisocyanate comprises a C₂-C₁₄ alkylene or cycloalkylene group, optionally, wherein:
(i) the C₂-C₁₄ alkylene group is linear; or
(ii) the C₂-C₁₄ alkylene group is branched; and/or
(b) the diisocyanate is 4,4'dicyclohexanemethane, 1'-transcyclohexane-diisocyanate, isophorone diisocyanate, 1,6-hexane diisocyanate, and/or 1,4-butane diisocyanate.

6. The hip labrum scaffold as in any preceding claim, wherein the polymer comprises a reaction product of a chain extender, optionally, wherein:
(a) the chain extender has the structure Y-R-Y, optionally, wherein:
(i) each Y is independently OH, NH₂, or NHR', optionally, wherein R' is a C₁-C₁₂ aliphatic group, optionally, wherein the aliphatic group is linear or is branched; and/or
(ii) both Y are OH; or
(iii) both Y are NH₂; and/or
(iv) R is a C₂-C₁₄ alkylene or cycloalkylene group.

7. The hip labrum scaffold as in any preceding claim, wherein the chain extender is:
(a) an ethylene diamine, a propylene diamine, a butane diamine, and/or a hexamethylene diamine; and/or
(b) 1,2-ethylene diamine and/or 1,6-hexamethylene diamine; and/or
(c) a cycloaliphatic diamine, optionally, wherein the cycloaliphatic diamine is 1,4-isophorone diamine and/or 1,4-cyclohexane diamine; and/or
(d) 1,4-butanediamine; and/or
(e) an aliphatic diol, optionally, wherein the aliphatic diol is ethylene glycol, diethylene glycol, dipropylene glycol, 1,4-butane diol, 1,6-hexane diol, 1,8-octane diol, neopentyl glycol, 1,12-dodecane diol, cyclohexane dimethanol, and/or 1,4-cyclohexane diol.

8. The hip labrum scaffold as in any preceding claim, wherein:
(a) a porosity of the porous foam is greater than or equal to 70% and less than or equal to 90%; and/or
(b) the porous foam comprises pores that are interconnected; and/or
(c) prior to implantation of the hip labrum scaffold in a patient, the hip labrum scaffold lacks biological materials; and/or
(d) a compressive modulus of the porous foam is greater than or equal to 300 kPa; and/or
(e) a tensile strength of the porous foam is greater than or equal to 600 kPa and less than or equal to 800 kPa; and/or
(f) a tensile modulus of the porous foam is greater than or equal to 500 kPa and less than or equal to 800 kPa; and/or
(g) a flexibility of the porous foam is greater than or equal to 100% and less than or equal to 500%; and/or
(h) a suture pull-out strength of the porous foam is greater than or equal to 2 N/mm and less than or equal to 5 N/mm; and/or
(i) the hip labrum scaffold, when subject to a compressive strain of greater than or equal to 60% retains at least 80% of its the open pores it had prior to application of the compressive strain; and/or
(j) a width of the hip labrum scaffold is greater than or equal to 2.5 mm and less than or equal to 5.5 mm; and/or
(k) a thickness of the hip labrum scaffold is greater than or equal to 2.5 mm and less than or equal to 8.5 mm; and/or
(l) catalysts make up less than or equal to 0.001 wt% of the polymer; and/or
(m) the hip labrum scaffold is positioned adjacent a patient's pelvis; and/or
(n) the hip labrum scaffold is positioned adjacent a patient's acetabulum; and/or
(o) when the hip labrum scaffold is positioned in a patient, the hip labrum scaffold is configured to fully degrade over a period of time of greater than or equal to 4 years and less than or equal to 6 years, optionally, wherein the degradation products are non-toxic.

9. The hip labrum scaffold as in any preceding claim, wherein the hip labrum scaffold:
(a) has a triangular cross-section; or
(b) has a round cross-section; or
(c) has a semicircular cross-section; or
(d) has a four-sided cross-section; or
(e) has a grooved cross-section, optionally, wherein:
(i) the groove is positioned proximate the bottom of the hip labrum scaffold; or
(ii) the groove is triangular; or
(iii) the groove is square; or
(f) is tubular; or
(g) has a donut-shaped cross-section.

10. The hip labrum scaffold as in any preceding claim, wherein the hip labrum scaffold is configured:
(a) to form a seal between a patient's acetabulum and the patient's femoral head; and/or
(b) to be implanted in a patient via an arthroscopic technique; and/or
(c) to be implanted in a patient via an open surgical technique; and/or
(d) to be implanted in a patient using a mini-open surgical technique.

11. A method for forming the porous foam of any preceding claims, wherein the method comprises:
preparing a solution of a synthetic polymer in a solvent, wherein the synthetic polymer makes up greater than or equal to 20 wt%/vol and less than or equal to 50 wt%/vol of the solution;
adding a nonsolvent for the synthetic polymer to the solution;
adding a pore-forming agent to the solution;
pouring the solution into a mold; and
washing the mold with a fluid in which the synthetic polymer is immiscible and in which the solvent, nonsolvent, and pore-forming agent are miscible, and, optionally:
(a) wherein the synthetic polymer makes up greater than or equal to 30 wt%/vol and less than or equal to 45 wt%/vol of the solution; and/or
(b) wherein the solvent comprises dimethylsulfoxide, dimethylformamide, chloroform, 1,4-dioxane, N-methylpyrrolidone, m-cresol, and/or dimethyl acetamide; and/or
(c) wherein the nonsolvent comprises water; and/or
(d) wherein the nonsolvent makes up greater than or equal to 5 vol% and less than or equal to 30 vol% of the volume of the solvent and the nonsolvent together; and/or
(e) wherein the nonsolvent makes up greater than or equal to 5 vol% and less than or equal to 10 vol% of the volume of the solvent and the nonsolvent together; and/or
(f) wherein the pore-forming agent is a sugar; and/or
(g) wherein the temperature is less than or equal to 10 °C above the melting point of the porous foam; and/or
(h) wherein the heating is performed for greater than or equal to 1 minute and less than or equal to 20 minutes.

12. The hip labrum scaffold of claims 1 to 10 for use in a method of hip labrum reconstruction, wherein the method comprises implanting said hip labrum scaffold in a patient.

## Patentansprüche

1. Hüftlabrumgerüst, umfassend:
einen porösen Schaumstoff, wobei:
der poröse Schaumstoff ein synthetisches Polymer umfasst; und
der poröse Schaumstoff eine durchschnittliche Porengröße von mehr als oder gleich 100 Mikrometer und weniger als oder gleich 400 Mikrometer aufweist,
wobei das Hüftlabrumgerüst so bemessen ist, dass es, wenn es in einem Patienten implantiert ist, einen geeigneten Ersatz für das Hüftlabrum darstellt.

2. Hüftlabrumgerüst nach Anspruch 1, wobei der poröse Schaumstoff geglüht wird, wobei das Glühen optional erreicht wird durch
Erhitzen des porösen Schaumstoffs auf eine Temperatur von mindestens 1 °C über seinem Schmelzpunkt, wodurch die durchschnittliche Porengröße des porösen Schaumstoffs erhöht wird.

3. Hüftlabrumgerüst nach Anspruch 1 oder 2, wobei das Polymer ein Polyurethan ist, und optional, wobei;
(a) das Polyurethan eine sich wiederholende aliphatische Einheit umfasst, wobei optional:
(i) die aliphatische sich wiederholende Einheit linear ist; oder
(ii) die aliphatische sich wiederholende Einheit verzweigt ist; und/oder
(b) das Polyurethan eine cycloaliphatische sich wiederholende Einheit umfasst.

4. Hüftlabrumgerüst nach einem vorstehenden Anspruch, wobei das Polymer:
(a) einen Polycaprolactonblock umfasst, wobei der Polycaprolactonblock optional ein Molekulargewicht von mehr als oder gleich 1 kDa und weniger als oder gleich 2 kDa aufweist; und/oder
(b) ein Reaktionsprodukt eines Makrodiols umfasst, wobei optional:
(i) das Makrodiol ein Reaktionsprodukt von ε-Caprolacton, Lactid, Glycolid, δ-Valerolacton, 1,4-Dioxan-2-on, 1,5-Dioxepan-2-on, Oxepan-2,7-dion, einem Polycarbonat, und/oder einem Copolycarbonat umfasst, wobei das Copolycarbonat optional ein Reaktionsprodukt von Trimethylencarbonat, Tetramethylencarbonat, 1,3-Dioxepan-2-on und/oder 1,3,8,10-Tetraoxacyclotetradecan umfasst; und/oder
(ii) das Makrodiol 1,6-Hexandiolpolycarbonat umfasst; und/oder
(iii) das Makrodiol ein Reaktionsprodukt eines C₁-C₁₀-Alkyldiols umfasst, wobei es sich bei dem C₁-C₁₀-Alkyldiol optional um 1,4-Butandiol handelt; und/oder
(c) ein zahlenmittleres Molekulargewicht von größer als oder gleich 100 kg/mol und kleiner als oder gleich 150 kg/mol aufweist; und/oder
(d) teilkristallin ist; und/oder
(e) einen Elastomerblock umfasst.

5. Hüftlabrumgerüst nach einem vorstehenden Anspruch, wobei das Polymer ein Reaktionsprodukt eines Diisocyanats umfasst, wobei optional:
(a) das Diisocyanat eine C₂-C₁₄-Alkylen- oder Cycloalkylengruppe umfasst, wobei optional:
(i) die C₂-C₁₄-Alkylengruppe linear ist; oder
(ii) die C₂-C₁₄-Alkylengruppe verzweigt ist; und/oder
(b) das Diisocyanat 4,4'-Dicyclohexanmethan, 1'-Transcyclohexan-Diisocyanat, Isophorondiisocyanat, 1,6-Hexandiisocyanat und/oder 1,4-Butandiisocyanat ist.

6. Hüftlabrumgerüst nach einem vorstehenden Anspruch, wobei das Polymer ein Reaktionsprodukt eines Kettenverlängerers umfasst, wobei optional:
(a) der Kettenverlängerer die Struktur Y-R-Y aufweist, wobei optional:
(i) jedes Y unabhängig OH, NH₂ oder NHR' ist, wobei optional R' eine aliphatische C₁-C₁₂-Gruppe ist, wobei optional die aliphatische Gruppe linear oder verzweigt ist; und/oder
(ii) beide Y OH sind; oder
(iii) beide Y NH₂ sind; und/oder
(iv) R eine C₂-C₁₄-Alkylen- oder Cycloalkylengruppe ist.

7. Hüftlabrumgerüst nach einem vorstehenden Anspruch, wobei der Kettenverlängerer Folgendes ist:
(a) ein Ethylendiamin, ein Propylendiamin, ein Butandiamin und/oder ein Hexamethylendiamin; und/oder
(b) 1,2-Ethylendiamin und/oder 1,6-Hexamethylendiamin; und/oder
(c) ein cycloaliphatisches Diamin, wobei das cycloaliphatische Diamin optional 1,4-Isophorondiamin und/oder 1,4-Cyclohexandiamin ist; und/oder
(d) 1,4-Butandiamin; und/oder
(e) ein aliphatisches Diol, wobei es sich bei dem aliphatischen Diol optional um Ethylenglykol, Diethylenglykol, Dipropylenglykol, 1,4-Butandiol, 1,6-Hexandiol, 1,8-Octandiol, Neopentylglykol, 1,12-Dodecandiol, Cyclohexandimethanol und/oder 1,4-Cyclohexandiol handelt.

8. Hüftlabrumgerüst nach einem vorstehenden Anspruch, wobei:
(a) eine Porosität des porösen Schaumstoffs größer als oder gleich 70 % und kleiner als oder gleich 90 % ist; und/oder
(b) der poröse Schaumstoff Poren umfasst, die miteinander verbunden sind; und/oder
(c) vor der Implantation des Hüftlabrumgerüsts in einen Patienten das Hüftlabrumgerüst keine biologischen Materialien enthält; und/oder
(d) ein Druckmodul des porösen Schaumstoffs größer als oder gleich 300 kPa ist; und/oder
(e) eine Zugfestigkeit des porösen Schaumstoffs größer als oder gleich 600 kPa und kleiner als oder gleich 800 kPa ist; und/oder
(f) ein Zugmodul des porösen Schaumstoffs größer als oder gleich 500 kPa und kleiner als oder gleich 800 kPa ist; und/oder
(g) eine Flexibilität des porösen Schaumstoffs größer als oder gleich 100 % und kleiner als oder gleich 500 % ist; und/oder
(h) eine Nahtausreißfestigkeit des porösen Schaumstoffs größer als oder gleich 2 N/mm und kleiner als oder gleich 5 N/mm ist; und/oder
(i) das Hüftlabrumgerüst, wenn es einer Druckbelastung von größer als oder 60 % ausgesetzt wird, mindestens 80 % seiner offenen Poren behält, die es vor der Anwendung der Druckbelastung hatte; und/oder
(j) eine Breite des Hüftlabrumgerüsts größer als oder gleich 2,5 mm und kleiner als oder gleich 5,5 mm ist; und/oder
(k) eine Dicke des Hüftlabrumgerüsts größer als oder gleich 2,5 mm und kleiner als oder gleich 8,5 mm ist; und/oder
(l) Katalysatoren weniger als oder gleich 0,001 Gew.% des Polymers ausmachen; und/oder
(m) das Hüftlabrumgerüst in der Nähe des Beckens eines Patienten positioniert ist; und/oder
(n) das Hüftlabrumgerüst in der Nähe der Hüftpfanne eines Patienten positioniert ist; und/oder
(o) wenn das Hüftlabrumgerüst in einem Patienten positioniert ist, das Hüftlabrumgerüst so konfiguriert ist, dass es sich über einen Zeitraum von mehr als oder gleich 4 Jahren und weniger als oder gleich 6 Jahren vollständig abbaut, wobei die Abbauprodukte optional nicht toxisch sind.

9. Hüftlabrumgerüst nach einem vorstehenden Anspruch, wobei das Hüftlabrumgerüst:
(a) einen dreieckigen Querschnitt aufweist; oder
(b) einen runden Querschnitt aufweist; oder
(c) einen halbkreisförmigen Querschnitt aufweist; oder
(d) einen vierseitigen Querschnitt aufweist; oder
(e) einen gerillten Querschnitt aufweist, wobei optional:
(i) die Rille in der Nähe des Bodens des Hüftlabrumgerüsts positioniert ist; oder
(ii) die Rille dreieckig ist; oder
(iii) die Rille quadratisch ist; oder
(f) röhrenförmig ist; oder
(g) einen donutförmigen Querschnitt aufweist.

10. Hüftlabrumgerüst nach einem vorstehenden Anspruch, wobei das Hüftlabrumgerüst konfiguriert ist:
(a) um eine Dichtung zwischen der Hüftpfanne eines Patienten und dem Oberschenkelkopf des Patienten zu bilden; und/oder
(b) um durch eine arthroskopische Technik in einen Patienten implantiert zu werden; und/oder
(c) um durch eine offene chirurgische Technik in einen Patienten implantiert zu werden; und/oder
(d) um mittels einer minimal-offenen chirurgischen Technik in einen Patienten implantiert zu werden.

11. Verfahren zur Bildung des porösen Schaumstoffs nach einem vorstehenden Anspruch, wobei das Verfahren Folgendes umfasst:
Herstellen einer Lösung eines synthetischen Polymers in einem Lösungsmittel, wobei das synthetische Polymer mehr als oder gleich 20 Gew.-%/Volumen und weniger als oder gleich 50 Gew.-%/Volumen der Lösung ausmacht;
Zugeben eines Nichtlösungsmittels für das synthetische Polymer zu der Lösung;
Zugeben eines porenbildenden Mittels zu der Lösung;
Gießen der Lösung in eine Form; und
Waschen der Form mit einem Fluid, in dem das synthetische Polymer nicht mischbar ist und in dem das Lösungsmittel, das Nichtlösungsmittel und das porenbildende Mittel mischbar sind, und optional:
(a) wobei das synthetische Polymer mehr als oder gleich 30 Gew.-%/Volumen und weniger als oder gleich 45 Gew.-%/Volumen der Lösung ausmacht; und/oder
(b) wobei das Lösungsmittel Dimethylsulfoxid, Dimethylformamid, Chloroform, 1,4-Dioxan, N-Methylpyrrolidon, m-Kresol und/oder Dimethylacetamid umfasst; und/oder
(c) wobei das Nichtlösungsmittel Wasser umfasst; und/oder
(d) wobei das Nichtlösungsmittel mehr als oder gleich 5 Vol% und weniger als oder gleich 30 Vol% des Volumens des Lösungsmittels und des Nichtlösungsmittels zusammen ausmacht; und/oder
(e) wobei das Nichtlösungsmittel mehr als oder gleich 5 Vol% und weniger als oder gleich 10 Vol% des Volumens des Lösungsmittels und des Nichtlösungsmittels zusammen ausmacht; und/oder
(f) wobei das porenbildende Mittel ein Zucker ist; und/oder
(g) wobei die Temperatur weniger als oder gleich 10 °C über dem Schmelzpunkt des porösen Schaumstoffs liegt; und/oder
(h) wobei das Erhitzen für mehr als oder gleich 1 Minute und weniger als oder gleich 20 Minuten durchgeführt wird.

12. Hüftlabrumgerüst nach einem der Ansprüche 1 bis 10 zur Verwendung in einem Verfahren zur Hüftlabrumrekonstruktion, wobei das Verfahren das Implantieren des Hüftlabrumgerüsts in einen Patienten umfasst.

## Revendications

1. Échafaudage de labrum de hanche, comprenant :
une mousse poreuse, dans laquelle :
la mousse poreuse comprend un polymère synthétique ; et
la mousse poreuse présente une taille de pore moyenne supérieure ou égale à 100 micromètres et inférieure ou égale à 400 micromètres,
dans laquelle l'échafaudage de labrum de hanche est dimensionné de telle sorte que, lorsqu'il est implanté dans un patient, il soit un remplacement approprié pour le labrum de hanche.

2. Échafaudage de labrum de hanche selon la revendication 1, dans lequel la mousse poreuse est recuite, dans lequel le recuit est facultativement réalisé en
chauffant la mousse poreuse à une température d'au moins 1 °C au-dessus de son point de fusion, augmentant ainsi la taille de pore moyenne de la mousse poreuse.

3. Échafaudage de labrum de hanche selon la revendication 1 ou revendication 2, dans lequel le polymère est un polyuréthane, et, facultativement, dans lequel :
(a) le polyuréthane comprend un motif de répétition aliphatique, facultativement, dans lequel :
(i) le motif de répétition aliphatique est linéaire ; ou
(ii) le motif de répétition aliphatique est ramifié ; et/ou
(b) le polyuréthane comprend un motif de répétition cycloaliphatique.

4. Échafaudage de labrum de hanche selon une quelconque revendication précédente, dans lequel le polymère :
(a) comprend un bloc de polycaprolactone, dans lequel facultativement, le bloc de polycaprolactone présente un poids moléculaire supérieur ou égal à 1 kDa et inférieur ou égal à 2 kDa ; et/ou
(b) comprend un produit de réaction d'un macrodiol, facultativement, dans lequel :
(i) le macrodiol comprend un produit de réaction de ε-caprolactone, lactide, glycolide, δ-valérolactone, 1,4-dioxane-2-one, 1,5-dioxepan-2-one, oxepan-2,7-dione, un polycarbonate et/ou un copolycarbonate, facultativement, dans lequel le copolycarbonate comprend un produit de réaction de triméthylènecarbonate, tétraméthylènecarbonate, 1,3-dioxepan-2-one et/ou 1,3,8,10-tétraoxacyclotétradécane ; et/ou
(ii) le macrodiol comprend du 1,6-hexanediol polycarbonate ; et/ou
(iii) le macrodiol comprend un produit de réaction d'un diol d'alkyle en C₁-C₁₀, facultativement, dans lequel le diol d'alkyle en C₁-C₁₀ est du 1,4-butanediol ; et/ou
(c) présente un poids moléculaire moyen en nombre supérieur ou égal à 100 kg/mol et inférieur ou égal à 150 kg/mol ; et/ou
(d) est semi-cristallin ; et/ou
(e) comprend un bloc élastomère.

5. Échafaudage de labrum de hanche selon une quelconque revendication précédente, dans lequel le polymère comprend un produit de réaction d'un diisocyanate, facultativement, dans lequel :
(a) le diisocyanate comprend un groupe alkylène ou cycloalkylène en C₂-C₁₄, facultativement, dans lequel :
(i) le groupe alkylène en C₂-C₁₄ est linéaire ; ou
(ii) le groupe alkylène en C₂-C₁₄ est ramifié ; et/ou
(b) le diisocyanate est du 4,4'dicyclohexaneméthane, du 1'-transcyclohexane-diisocyanate, de l'isophorone diisocyanate, du 1,6-hexane diisocyanate et/ou du 1,4-butane diisocyanate.

6. Échafaudage de labrum de hanche selon une quelconque revendication précédente, dans lequel le polymère comprend un produit de réaction d'un allongeur de chaîne, facultativement, dans lequel :
(a) l'allongeur de chaîne présente la structure Y-R-Y, facultativement, dans lequel :
(i) chaque Y est indépendamment OH, NH₂ ou NHR', facultativement, dans lequel R' est un groupe aliphatique en C₁-C₁₂, facultativement, dans lequel le groupe aliphatique est linéaire ou ramifié ; et/ou
(ii) les deux Y sont OH ; ou
(iii) les deux Y sont NH₂ ; et/ou
(iv) R est un groupe alkylène ou cycloalkylène en C₂-C₁₄.

7. Échafaudage de labrum de hanche selon une quelconque revendication précédente, dans lequel l'allongeur de chaîne est :
(a) une diamine d'éthylène, une diamine de propylène, une diamine de butane et/ou une diamine d'hexaméthylène ; et/ou
(b) de la diamine de 1,2-éthylène et/ou de la diamine de 1,6-hexaméthylène ; et/ou
(c) une diamine cycloaliphatique, facultativement, dans lequel la diamine cycloaliphatique est de la diamine de 1,4-isophorone et/ou de la diamine de 1,4-cyclohexane ; et/ou
(d) de la 1,4-butanediamine ; et/ou
(e) un diol aliphatique, facultativement, dans lequel le diol aliphatique est de l'éthylène glycol, du diéthylène glycol, du dipropylène glycol, du 1,4-butane diol, du 1,6-hexane diol, du 1,8-octane diol, du néopentyl glycol, du 1,12-dodécane diol, du diméthanol de cyclohexane et/ou du 1,4-cyclohexane diol.

8. Échafaudage de labrum de hanche selon une quelconque revendication précédente, dans lequel :
(a) une porosité de la mousse poreuse est supérieure ou égale à 70 % et inférieure ou égale à 90 % ; et/ou
(b) la mousse poreuse comprend des pores qui sont reliés entre eux ; et/ou
(c) avant implantation de l'échafaudage de labrum de hanche dans un patient, l'échafaudage de labrum de hanche est dépourvu de matières biologiques ; et/ou
(d) un module de compression de la mousse poreuse est supérieur ou égal à 300 kPa ; et/ou
(e) une résistance à la traction de la mousse poreuse est supérieure ou égale à 600 kPa et inférieure ou égale à 800 kPa ; et/ou
(f) un module de traction de la mousse poreuse est supérieur ou égal à 500 kPa et inférieur ou égal à 800 kPa ; et/ou
(g) une flexibilité de la mousse poreuse est supérieure ou égale à 100 % et inférieure ou égale à 500 % ; et/ou
(h) une résistance à l'arrachement des sutures de la mousse poreuse est supérieure ou égale à 2 N/mm et inférieure ou égale à 5 N/mm ; et/ou
(i) l'échafaudage de labrum de hanche, lorsqu'il est soumis à une contrainte de compression supérieure ou égale à 60 % conserve au moins 80 % de ses pores ouverts qu'il avait avant l'application de la contrainte de compression ; et/ou
(j) une largeur de l'échafaudage de labrum de hanche est supérieure ou égale à 2,5 mm et inférieure ou égale à 5,5 mm ; et/ou
(k) une épaisseur de l'échafaudage de labrum de hanche est supérieure ou égale à 2,5 mm et inférieure ou égale à 8,5 mm ; et/ou
(l) des catalyseurs représentent 0,001 % en poids ou moins du polymère ; et/ou
(m) l'échafaudage de labrum de hanche est positionné adjacent au pelvis d'un patient ; et/ou
(n) l'échafaudage de labrum de hanche est positionné adjacent à l'acétabulum d'un patient ; et/ou
(o) lorsque l'échafaudage de labrum de hanche est positionné dans un patient, l'échafaudage de labrum de hanche est configuré pour se dégrader entièrement sur une période de temps de 4 ans ou plus et de 6 ans ou moins, facultativement, dans lequel les produits de dégradation sont non toxiques.

9. Échafaudage de labrum de hanche selon une quelconque revendication précédente, dans lequel l'échafaudage de labrum de hanche :
(a) présente une section transversale triangulaire ; ou
(b) présente une section transversale ronde ; ou
(c) présente une section transversale semi-circulaire ; ou
(d) présente une section transversale à quatre côtés ; ou
(e) présente une section transversale rainurée, facultativement, dans laquelle :
(i) la rainure est positionnée à proximité du fond de l'échafaudage de labrum de hanche ; ou
(ii) la rainure est triangulaire ; ou
(iii) la rainure est carrée ; ou
(f) est tubulaire ; ou
(g) présente une section transversale en forme de beignet.

10. Échafaudage de labrum de hanche selon une quelconque revendication précédente, dans lequel l'échafaudage de labrum de hanche est configuré :
(a) pour former un joint entre l'acétabulum d'un patient et la tête fémorale du patient ; et/ou
(b) pour être implanté dans un patient via une technique arthroscopique ; et/ou
(c) pour être implanté dans un patient via une technique chirurgicale ouverte ; et/ou
(d) pour être implanté dans un patient en utilisant une mini-technique chirurgicale ouverte.

11. Procédé de formation de la mousse poreuse selon une quelconque revendication précédente, dans lequel le procédé comprend :
la préparation d'une solution d'un polymère synthétique dans un solvant, dans lequel le polymère synthétique représente 20 % en poids ou plus et 50 % en poids ou moins de la solution ;
l'ajout à la solution d'un non-solvant pour le polymère synthétique ;
l'ajout à la solution d'un agent de formation de pores ;
le versement de la solution dans un moule ; et
le lavage du moule à l'aide d'un fluide dans lequel le polymère synthétique est immiscible et dans lequel le solvant, le non-solvant et l'agent de formation de pores sont miscibles, et, facultativement :
(a) dans lequel le polymère synthétique représente 30 % en poids ou plus et 45 % en poids ou moins de la solution ; et/ou
(b) dans lequel le solvant comprend du diméthylsulfoxyde, du diméthylformamide, du chloroforme, du 1,4-dioxane, du N-méthylpyrrolidone, du m-crésol et/ou de l'acétamide de diméthyle ; et/ou
(c) dans lequel le non-solvant comprend de l'eau ; et/ou
(d) dans lequel le non-solvant représente 5 % en volume ou plus et 30 % en volume ou moins du volume du solvant et du non-solvant ensemble ; et/ou
(e) dans lequel le non-solvant représente 5 % en volume ou plus et 10 % en volume ou moins du volume du solvant et du non-solvant ensemble ; et/ou
(f) dans lequel l'agent de formation de pores est un sucre ; et/ou
(g) dans lequel la température est inférieure ou égale à 10 °C au-dessus du point de fusion de la mousse poreuse ; et/ou
(h) dans lequel le chauffage est mis en œuvre pendant 1 minute ou plus et 20 minutes ou moins.

12. Échafaudage de labrum de hanche selon les revendications 1 à 10 pour utilisation dans un procédé de reconstruction de labrum de hanche, dans lequel le procédé comprend l'implantation dudit échafaudage de labrum de hanche dans un patient.
